# EUROPEAN PATENT APPLICATION

(11) **EP 4 276 093 A1**
(43) Date of publication of application: **15.11.2023**
(21) Application number: 22382445.9
(22) Date of filing: 09.05.2022
(51) Int. Cl.: C07D 209/08, C07D 401/12, C07D 403/12, C07D 405/12, A61P 31/12

(54) **ANTIVIRAL COMPOUNDS AND COMPOSITIONS THEREFROM**

(71) Applicant: Consejo Superior De Investigaciones Científicas, 28006 Madrid (ES)
(72) Inventor: GUTIÉRREZ RODRÍGUEZ, Marta, 28006 Madrid (ES); HERRANZ HERRANZ, Rosario, 28006 Madrid (ES); GASTAMINZA LANDART, Pablo, 28006 Madrid (ES); GARAIGORTA DE DIOS, Urtzi, 28006 Madrid (ES); CASTRO NAVAS, Francisco F., 28006 Madrid (ES); CABRERA TORREJÓN, Daniel, 28006 Madrid (ES); CASTRO ILLANA, Victoria, 28006 Madrid (ES); BUENO FERNÁNDEZ, Paula, 28006 Madrid (ES)
(74) Representative: ABG Intellectual Property Law, S.L.

(57) **Abstract**

The present invention relates to a compound of formula (I) or a pharmaceutically acceptable salt thereof. It also relates to a compound of formula (I) or a pharmaceutically acceptable salt thereof for use in medicine, in particular for the prevention of treatment of infection by coronavirus. The invention also relates to a process for the preparation of a compound of formula (I) or a pharmaceutically acceptable salt thereof

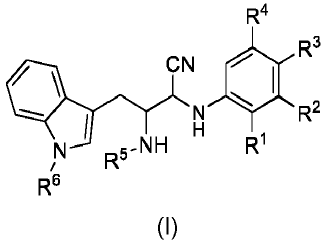

## Description

### FIELD OF THE INVENTION

The present invention relates to 2,3-diamino-4-(1*H*-indol-3-yl)butanenitrile compounds of formula (I) or pharmaceutically acceptable salts thereof. It also relates to compounds of formula (I) or pharmaceutically acceptable salts thereof for use in medicine, in particular for the prevention or treatment of infection by coronavirus. The invention also relates to a process for the preparation of compounds of formula (I) or pharmaceutically acceptable salts thereof.

### BACKGROUND

Different types of coronavirus affecting human beings are known in the art. These viruses are characterized for having a corona-shaped spike on their surface. Common coronaviruses are 229E (alpha coronavirus), NL63 (alpha coronavirus), OC43 (beta coronavirus) and HKU1 (beta coronavirus). Such viruses commonly infect human beings. Certain coronaviruses affecting human beings come from the evolution of animal coronavirus. Such viruses may provoke more severe conditions, such as respiratory syndromes. Recent examples of such coronaviruses are MERS-CoV (the beta coronavirus that causes Middle East Respiratory Syndrome, or MERS), SARS-CoV (the beta coronavirus that causes severe acute respiratory syndrome, or SARS) and SARS-CoV-2 (the coronavirus that caused coronavirus disease 2019, or COVID-19).

Because most coronaviruses are new, the degree of immunity to such viruses still remain low, so that these coronaviruses have the potential to infect a large number of people. Despite the incidence of severe cases with acute respiratory syndromes is rather low, the large potential for global contamination provides for a potential to generate a large number of cases with severe symptoms. Consequently, there is a need for anti-infective agents allowing for preventing or treating infection with coronaviruses.

Several agents suitable for the treatment or prevention of diseases provoked by coronavirus infection are known in the art. For instance, remdesivir was shown to interfere in RNA polymerization of SARS-coronavirus and MERS-coronavirus in vitro, thereby being suitable for preventing the development of severe forms of coronavirus disease infection. Remdesivir has been approved by the US Food and Drug Administration for the treatment of infection by SARS-Cov-2.

Other anti-infective agents are known in the art. For instance, it has been reported as well that the combination of nirmatrelvir and ritonavir (commercialized with the name paxlovid) prevents the reproduction of the virus by inhibition of an enzyme. Other small molecules with anti-infective properties against coronavirus infection include ivermectin, which is a mixture of avermectins B1a and B1b, molnupiravir and nitazoxadin.

Certain derivatives of 2,3-diamino-4-(1*H*-indol-3-yl)butanenitrile derivatives are known in the art. For instance, Herrero, S. et al., Journal of Organic Chemistry (2003), 68(11), 4582-4585 reports the compound of formula (11) as a 2:1 mixture of (*S*,*S*) and (S,R) diastereomers

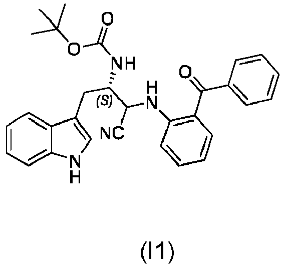

These compounds are disclosed as synthetic intermediates in the synthesis of 5-phenyl-1,4-benzodiazepine derivatives. The authors are however silent about the use of these compounds of formula (11) in medicine or about any biological activity for said compounds.

Additionally, Herrero S. et al., Tetrahedron (2003), 59(25), 4491-4499 discloses the compound of formula (I2)

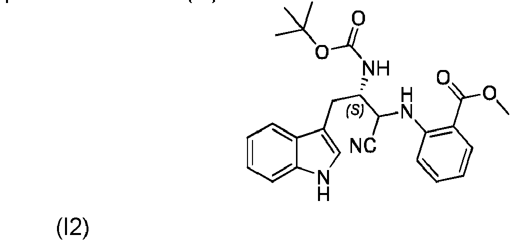

This compound is disclosed as synthetic intermediate in the synthesis of chiral 2-substituted-5-oxo-1,2,3,4-tetrahydro-5*H*-1,4-benzodiazepines. The authors are however silent about the use of the compound of formula (12) in medicine or about any biological activity for said compound.

From what is disclosed in the art, it derives that there is still a need for the provision of small molecules with anti-viral activity, in particular for use in the treatment or prevention of infection with coronavirus.

### SUMMARY OF THE INVENTION

After exhaustive research, the inventors have found that certain 2,3-diamino-4-(1*H*-indol-3-yl)butanenitrile derivatives unexpectedly exhibit anti-viral activity and are useful for the treatment or prevention of infection with coronavirus.

Thus, in a first aspect, the invention relates to a compound of formula (I) or a pharmaceutically acceptable salt thereof 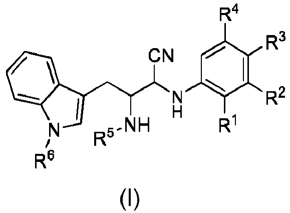 wherein R¹ is a group selected from the group consisting of hydrogen, halogen, carboxyl (-COOH), (C₁-C₄)alkyloxycarbonyl, (C₁-C₄)alkylcarbonyl, phenylcarbonyl, and a group of formula -CONH-(CH₂)ₙ-Ph wherein n is 0 or 1;
R² is a group selected from the group consisting of hydrogen, halogen, (C₁-C₄)alkyloxycarbonyl, and phenylcarbonyl;
R³ is a group selected from the group consisting of:
   a) hydrogen,
   b) halogen,
   c) (C₁-C₄)alkyl,
   d) (C₁-C₄)alkyloxycarbonyl, and
   e) a group selected from the group consisting of pyridinyl, pyrimidinyl and phenyl, said group being optionally substituted at any available position with one or two groups selected from the group consisting of halogen, (C₁-C₄)alkyl, (C₁-C₄)alkyloxy, trilfuoromethyloxy, di((C₁-C₄)alkyl)amino, phenyl, benzyl, (C₃-C₆)cycloalkyl and a 5 to 6-membered saturated heterocyclic ring, the members of the ring being selected from the group consisting of C, CH, CH₂, O, N and NH, said ring being optionally substituted at any available position with a (C₁-C₄)alkyl; or, alternatively, wherein said group selected from the group consisting of pyridinyl, pyrimidinyl and phenyl optionally forms a 6-membered ring with a divalent group of formula -O-(CH₂)₂-O- whereby the oxygen atoms of said divalent group are connected to two adjacent carbon atoms of said pyridinyl, pyrimidinyl or phenyl group;
R⁴ is a group selected from the group consisting of hydrogen, halogen and phenyl optionally substituted at any available position with a (C₁-C₄)alkyl group;
R⁵ and R⁶ are each a group independently selected from the group consisting of hydrogen and a group of formula C₁₋₄-alkyl-(O)ₓ-CO-, wherein x has a value of 0 or 1 and wherein the C₁₋₄-alkyl group is optionally substituted with 1 to 3 halogen groups;
with the proviso that the compound of formula (I) is not a compound of formula (11) or (12)

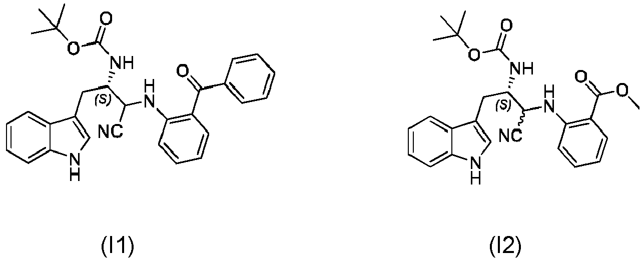

The compound of the first aspect of the invention may be comprised in a pharmaceutical composition. A second aspect of the invention thus relates to a composition comprising a compound of formula (I) or a compound of formula (11) or a compound of formula (12) as defined in the first aspect of the invention, or a pharmaceutically acceptable salt thereof and one or more pharmaceutically acceptable diluent or carrier.

The compounds of formulae (I), (11) and (12) are useful in the prevention or treatment of infection with coronavirus. A third aspect of the invention relates to a compound of formula (I) or a compound of formula (11), or a compound of formula (12) as defined in the first aspect of the invention, or a pharmaceutically acceptable salt thereof, or a composition as defined in the second aspect of the invention, for use in medicine.

A fourth aspect of the invention relates to a compound of formula (I) or a compound of formula (11), or a compound of formula (12) as defined in the first aspect of the invention, or a pharmaceutically acceptable salt thereof, or a composition as defined in the second aspect of the invention, for use in the treatment or prevention of viral infection by coronavirus.

A fifth aspect of the invention relates to a process for the preparation of a compound of formula (I) as defined in the first aspect of the invention, comprising the steps of:
(a) contacting a compound of formula (II) with a compound of formula (III) 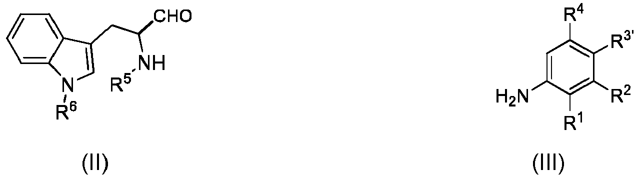 in order to form a compound of formula (IV) 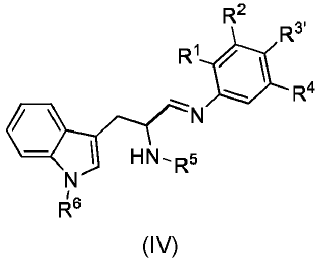 wherein R¹, R², R⁴, R⁵ and R⁶ are as defined in the first aspect of the invention,
   R^{3'} receives the same definition as the R³ group in the first aspect of the invention, and
(b) treating the product of step (a) with a cyanide source in order to form a product of formula (I).

### BRIEF DESCRIPTION OF THE DRAWINGS

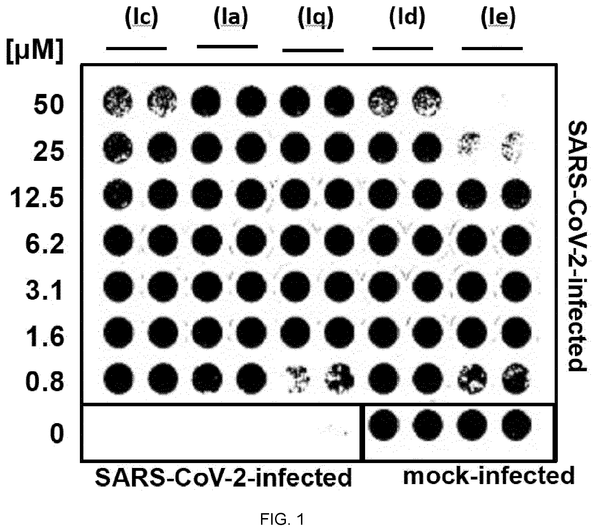

Fig. 1 shows the resistance of a cell monolayer population infected with SARS-Cov2 and treated or not treated with a compound of formula (I) at variable concentrations, according to the procedure of Example 3. Compounds displaying protective activity are identified as compounds for which positive staining, similar to mock-infected cells, is observed in cells inoculated with the virus.

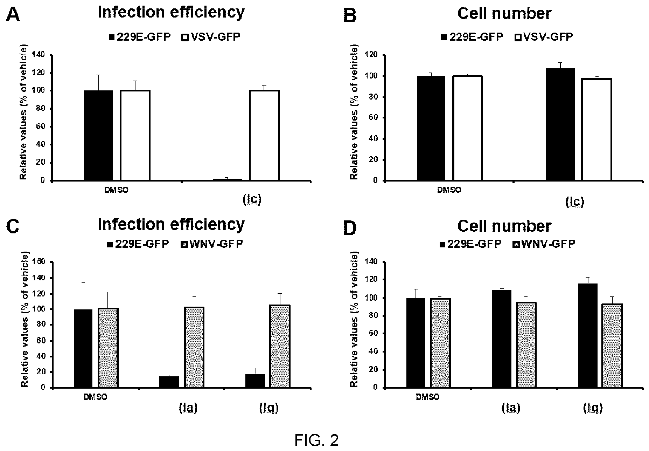

Fig. 2 shows viral propagation within cell populations treated with the compounds (Ia), (Ic) or (Iq) and infected with 229E-GFP coronavirus strain (panels A and C), vesicular stomatitis virus expressing GFP (VSV-GFP, panel A) or West Nile Virus expressing GFP (WNV-GFP, panel C); panels B and D represent cell biomass as determined by DAPI staining to verify the absence of cytotoxicity.

### DETAILED DESCRIPTION

All terms as used herein in this application, unless otherwise stated, shall be understood in their ordinary meaning as known in the art. Other more specific definitions for certain terms as used in the present application are as set forth below and are intended to apply uniformly throughout the specification and claims unless an otherwise expressly set out definition provides a broader definition.

For the purposes of the invention, any ranges given include both the lower and the upper end-points of the range. Ranges given, such as temperatures, times, molar ratio, volume ratio and the like, should be considered approximate (i.e. with a 5% margin of variation around indicated point), unless specifically stated.

In the context of the invention, the term "alkyl" refers to an aliphatic saturated hydrocarbon chain that is linear or branched and having the number of carbon atoms defined in the claims and the description. Non-limiting examples of alkyl groups include, for instance, methyl, ethyl, n-propyl, *i*-propyl, n-butyl, *i*-butyl, *t*-butyl, n-pentyl, neo-pentyl, hexyl, heptyl, octyl, nonyl, decyl, undecanyl and dodecanyl.

In the context of the invention, the term "halogen" or "halo" refers to a halogen group, such as chloro, bromo, fluoro and iodo.

In the context of the invention, the term "cycloalkyl" refers to an aliphatic saturated hydrocarbon cycle and having the number of carbon atoms defined in the claims and the description. Non-limiting examples of cycloalkyl groups include, for instance, cyclopropyl, cyclobutyl, cyclopentyl and cyclohexyl.

In the context of the invention, the term "haloalkyl" refers to an alkyl group as defined above and having the number of carbon atoms defined in the claims and the description whereby at least one of the hydrogen atoms is replaced with a halo group. Thus, non-limiting examples of haloalkyl groups include, among others, chloromethyl, fluoromethyl, perfluoroalkyl groups such as trifluoromethyl, tetrafluoroethyl, and bromomethyl.

In the context of the invention, the term "alkylcarbonyl" refers to a saturated linear or branched hydrocarbon group having the number of carbon atoms indicated in the description or in the claims which is attached to the remainder of the molecular formula through a carbonyl group (C=O).

In the context of the invention, the term "alkylcarbonyloxy" refers to a saturated linear or branched hydrocarbon group having the number of carbon atoms indicated in the description or in the claims which is attached to the remainder of the molecular formula through a carboxyl group (-COO-) and wherein the alkyl chain is attached to the carbon atom of the carboxyl group.

In the context of the invention, the term "alkyloxy" refers to a saturated linear or branched hydrocarbon group having the number of carbon atoms indicated in the description or in the claims which is attached to the remainder of the molecular formula through a oxy group (-O-) and wherein the alkyl chain is attached to the oxygen atom of the oxy group.

In the context of the invention, the term "alkyloxycarbonyl" refers to a saturated linear or branched hydrocarbon group having the number of carbon atoms indicated in the description or in the claims which is attached to the remainder of the molecular formula through a carboxyl group (-OOC-) and wherein the alkyl chain is attached to the oxygen atom of the carboxyl group.

In the context of the invention, the term "catalytically effective amount" refers to the fact that the amount of catalyst is much smaller than the stoichiometric amounts of either starting materials of a catalytic reaction and is sufficient for the catalyzed process to take place at a reaction rate that is at least twice the rate of the non- catalyzed process.

As defined above, a first aspect of the invention relates to a compound of formula (I) as defined above in the first aspect of the invention. In a preferred embodiment of the first aspect of the invention, the compound of formula (I) is as defined in the first aspect of the invention with the proviso that the compound of formula (I) is not a compound of formula (11), (12) or (13)

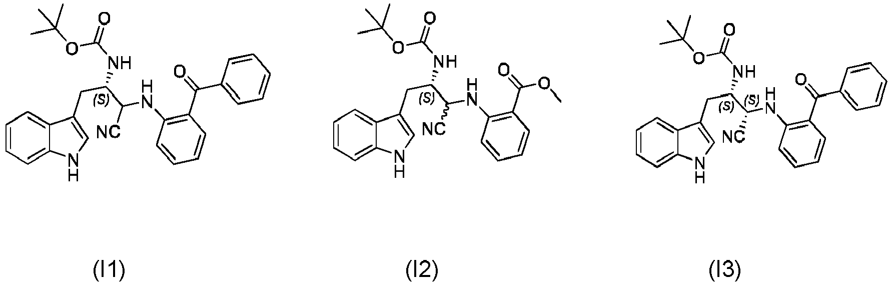

The compound of formula (I) may exist in different diastereomeric forms, in particular, the following diastereomeric forms (I'a), (I'b), (I"a) and (I"b) are contemplated:

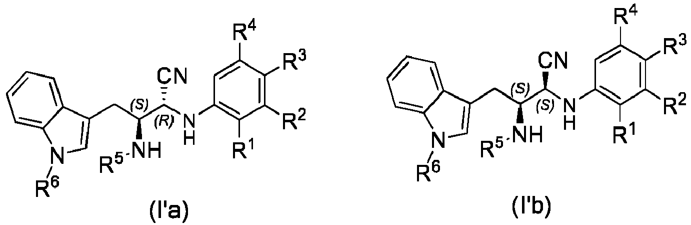

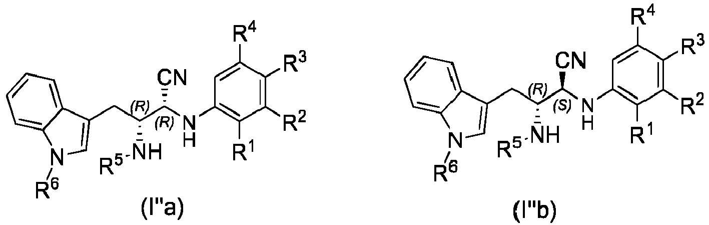

As disclosed herein, when the configuration of a stereocenter in the compound of formula (I) is not specified or is represented by a coplanar bond, it refers to the fact that said stereocenter may be in either (R) or (S) configuration. Thus, formula (I) with non-specified configuration of the two stereocenters is intended to cover the 4 possible stereoisomers *(R,R), (R,S), (S,R)* and (S,S) or any mixture thereof. In a particular embodiment the compound may exist as a mixture of two compounds whereby, in the first compound, said stereocenter is in the (R) configuration and, in the second compound, said stereocenter is in the (S) configuration.

In a preferred embodiment of the first aspect of the invention, the compound of formula (I) is one of formula (I') or a pharmaceutically acceptable salt thereof

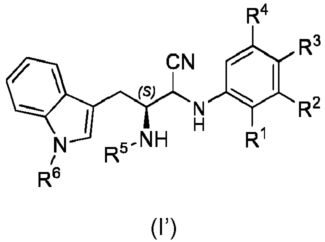

The compound of formula (I') thus represents a compound of formula (I'a) as defined above or a compound of formula (I'b) as defined above or a mixture thereof.

In a more preferred embodiment of the first aspect of the invention, the compound of formula (I) is a mixture of the diastereomeric forms (I'a) and (I'b) as defined above.

In particular embodiments of the first aspect of the invention, when the compound of formula (I) is a mixture of the diastereomeric forms (I'a) and (I'b) as defined above, the molar ratio of the compound of formula (I'a) to the compound of formula (I'b) is comprised from 1:10 to 10:1.

In particular embodiments of the first aspect of the invention, when the compound of formula (I) is a mixture of the diastereomeric forms (I'a) and (I'b) as defined above, the molar ratio of the compound of formula (I'a) to the compound of formula (I'b) is comprised from 1:5 to 10:1.

In more particular embodiments of the first aspect of the invention, when the compound of formula (I) is a mixture of the diastereomeric forms (I'a) and (I'b) as defined above, the molar ratio of the compound of formula (I'a) to the compound of formula (I'b) is selected from the group consisting of 9:1, 2:1, 1:1, 1:1.3, 1:1.5, 1:2, 1:3 and 1:5.

In other preferred embodiments of the first aspect of the invention, the compound of formula (I) is one wherein R¹ is a group selected from the group consisting of hydrogen, halogen, (C₁-C₄)alkyloxycarbonyl, (C₁-C₄)alkylcarbonyl, phenylcarbonyl, and a group of formula -CONH-(CH₂)ₙ-Ph wherein n is 0 or 1.

In a more preferred embodiment of the first aspect of the invention, the compound of formula (I) is one wherein R¹ is a group selected from the group consisting of hydrogen, bromide, methyloxycarbonyl, ethyloxycarbonyl, *tert*-butyloxycarbonyl, methylcarbonyl, phenylcarbonyl, and a group of formula -CONH-(CH₂)ₙ-Ph wherein n is 0 or 1.

In another preferred embodiment of the first aspect of the invention, the compound of formula (I) is one wherein R² is a group selected from the group consisting of hydrogen, bromide, methyloxycarbonyl, ethyloxycarbonyl and phenylcarbonyl.

In a more preferred embodiment of the first aspect of the invention, the compound of formula (I) is one wherein R² is hydrogen or bromide.

In another preferred embodiment of the first aspect of the invention, the compound of formula (I) is one wherein R³ is hydrogen.

In another preferred embodiment of the first aspect of the invention, the compound of formula (I) is one wherein R³ is halogen; preferably, R³ is bromo or chloro.

In another preferred embodiment of the first aspect of the invention, the compound of formula (I) is one wherein R³ is (C₁-C₄)alkyloxycarbonyl; preferably, R³ is methyloxycarbonyl.

In another preferred embodiment of the first aspect of the invention, the compound of formula (I) is one wherein R³ is a group selected from the group consisting of pyridinyl, pyrimidinyl and phenyl, wherein said group optionally forms a 6-membered ring with a divalent group of formula -O-(CH₂)₂-O- whereby the oxygen atoms of said divalent group are connected to two adjacent carbon atoms of said pyridinyl, pyrimidinyl or phenyl group.

In a more particular embodiment of the first aspect of the invention, the compound of formula (I) is one wherein R³ is 2,3-dihydro-benzo[1,4]dioxin-6-yl.

In another preferred embodiment of the first aspect of the invention, the compound of formula (I) is one wherein R³ is a radical deriving from pyridine, pyrimidine or benzene optionally substituted at any available position with one or two groups selected from the group consisting of halogen, (C₁-C₄)alkyl, (C₁-C₄)alkyloxy, trilfuoromethyloxy, di((C₁-C₄)alkyl)amino, phenyl, benzyl, (C₃-C₆)cycloalkyl and a 5 to 6-membered saturated heterocyclic ring, the members of the ring being selected from the group consisting of C, CH, CH₂, O, N and NH, said ring being optionally substituted at any available position with a (C₁-C₄)alkyl.

In another preferred embodiment of the first aspect of the invention, the compound of formula (I) is one wherein R³ is a group selected from the group consisting of pyridinyl, pyrimidinyl and phenyl optionally substituted at any available position with one or two groups selected from the group consisting of halogen, (C₁-C₄)alkyl, (C₁-C₄)alkyloxy, trilfuoromethyloxy, di((C₁-C₄)alkyl)amino, phenyl, benzyl, cyclopropyl and a 5 to 6-membered saturated heterocyclic ring selected from the group consisting of pyrrolidinyl, piperazinyl, morpholinyl and piperidinyl, said ring being optionally substituted at any available position with a methyl or isopropyl group.

In another preferred embodiment of the first aspect of the invention, the compound of formula (I) is one wherein R³ is a group selected from the group consisting of pyridinyl, pyrimidinyl and phenyl optionally substituted at any available position with one or two groups selected from the group consisting of halogen, (C₁-C₄)alkyl, (C₁-C₄)alkyloxy, trilfuoromethyloxy, di((C₁-C₄)alkyl)amino, phenyl, benzyl, cyclopropyl and a 5 to 6-membered saturated heterocyclic ring selected from the group consisting of pyrrolidinyl, piperazinyl and morpholinyl, said ring being optionally substituted at any available position with a methyl or isopropyl group.

In a more preferred embodiment of the first aspect of the invention, the compound of formula (I) is one wherein R³ is a group selected from the group consisting of pyridinyl, pyrimidinyl and phenyl optionally substituted at any available position with one or two groups selected from the group consisting of halogen, (C₁-C₄)alkyl, (C₁-C₄)alkyloxy, trilfuoromethyloxy, di((C₁-C₄)alkyl)amino, phenyl, benzyl, cyclopropyl and a 5 to 6-membered saturated heterocyclic ring selected from the group consisting of pyrrolidinyl, piperazinyl, morpholinyl and piperidinyl, said ring being optionally substituted at any available position with a methyl or isopropyl group.

In a more preferred embodiment of the first aspect of the invention, the compound of formula (I) is one wherein R³ is a group selected from the group consisting of pyridinyl, pyrimidinyl and phenyl optionally substituted at any available position with one or two groups selected from the group consisting of fluoro, n-butyl, *tert-butyl, iso*-propyl, ethyl, methyl, methoxy, ethoxy, isopropyloxy, trilfuoromethyloxy, diethylamino, dimethylamino, phenyl, benzyl, cyclopropyl and a 5 to 6-membered saturated heterocyclic ring selected from the group consisting of pyrrolidinyl, piperazinyl and morpholinyl, said ring being optionally substituted at any available position with a methyl or isopropyl group.

In a more preferred embodiment of the first aspect of the invention, the compound of formula (I) is one wherein R³ is a group selected from the group consisting of hydrogen, bromide, chloride, methyloxycarbonyl, 4-(*N*-morpholinyl)phenyl, 3-(N-morpholinyl)phenyl, 4-tert-butylphenyl, 4-ethylphenyl, 4-cyclopropylphenyl, 2,3-dihydro-benzo[1,4]dioxin-6-yl, 4-(4-methylpiperazin-1-yl)phenyl, 4-butylphenyl, 3-benzylphenyl, 2-fluoro-5-isopropyloxyphenyl, 6-pyrrodinylpyridin-3-yl, 6-methylpyridin-3-yl, 6-(N-morpholinyl)pyridine-3-yl, 6-methoxypyridin-3-yl, 6-ethoxypyrimidin-3-yl, 4-diethylaminophenyl, 4-propyloxyphenyl, 3-tert-butylphenyl, 3-ethylphenyl, 3-pyrrolidinylphenyl, 3-diethylaminophenyl, 3-trifluoromethoxyphenyl, and 6-(4-isopropylpiperazin-1-yl)pyridin-3-yl.

In another preferred embodiment of the first aspect of the invention, the compound of formula (I) is one wherein R⁴ is a group selected from the group consisting of hydrogen, bromide and 4-*tert*-butylphenyl.

In another preferred embodiment of the first aspect of the invention, the compound of formula (I) is one wherein R⁴ is hydrogen.

In another preferred embodiment of the first aspect of the invention, the compound of formula (I) is one wherein R⁵ is selected from the group consisting of hydrogen, *tert*-butyloxycarbonyl, pivaloyl and trifluoromethylcarbonyl. More preferably, R⁵ is selected from the group consisting of *tert*-butyloxycarbonyl.

In another preferred embodiment of the first aspect of the invention, the compound of formula (I) is one wherein R⁶ is selected from the group consisting of hydrogen, *tert*-butyloxycarbonyl, pivaloyl and trifluoromethylcarbonyl. More preferably, R⁶ is hydrogen.

The different groups R¹, R², R³, R⁴, R⁵ and R⁶ defined in the particular and preferred embodiments above may be combined to provide compounds of formula (I) according to the first aspect of the invention.

Particularly, the compound of the first aspect of the invention is selected from the group consisting of:
methyl 4-(((2*S*)-2-((*tert*-butoxycarbonyl)amino)-1-cyano-3-(1*H*-indol-3-yl)propyl)-amino)-4'-morpholino-[1,1'-biphenyl]-3-carboxylate;
methyl 5-bromo-2-(((2*S*)-2-((*tert*-butoxycarbonyl)amino)-1-cyano-3-(1*H*-indol-3-yl)propyl)amino)benzoate;
(3*S*)-2-((4-Bromo-2-benzoylphenyl)amino)-4-(1*H*-indol-3-yl)-3-(*tert-*butoxycarbonylamino) butanenitrile;
(3*S*)-3-(*tert*-Butoxycarbonylamino)-4-(1*H*-indol-3-yl)-2-((2-benzoyl-4-chlorophenyl)amino) butanenitrile;
methyl 5-bromo-2-(((2*S*)-2-((*tert*-butoxycarbonyl)amino)-1-cyano-3-(1*H*-indol-3-yl)propyl)amino)benzoate;
methyl 2-(((2*S*)-2-((*tert*-butoxycarbonyl)amino)-1-cyano-3-(1*H*-indol-3-yl)propyl)amino)-5-(2,3-dihydrobenzo[*b*][1,4]dioxin-6-yl)benzoate;
methyl 4-(((2*S*)-2-((*tert*-butoxycarbonyl)amino)-1-cyano-3-(1*H*-indol-3-yl)propyl)amino)-3'-morpholino-[1,1'-biphenyl]-3-carboxylate;
ethyl 2-(((2*S*)-2-((*tert*-butoxycarbonyl)amino)-1-cyano-3-(1*H*-indol-3-yl)propyl)amino)-benzoate;
methyl 4-bromo-2-(((2*S*)-2-((*tert*-butoxycarbonyl)amino)-1-cyano-3-(1*H*-indol-3-yl)propyl)-amino)benzoate;
methyl 4-(((2*S*)-2-((*tert*-butoxycarbonyl)amino)-1-cyano-3-(1*H*-indol-3-yl)propyl)amino)-4'-(*tert*-butyl)-[1,1'-biphenyl]-3-carboxylate;
methyl 2-bromo-6-(((2*S*)-2-((*tert*-butoxycarbonyl)amino)-1-cyano-3-(1*H*-indol-3-yl)propyl)-amino)benzoate;
methyl 4-(((2*S*)-2-((*tert*-butoxycarbonyl)amino)-1-cyano-3-(1*H*-indol-3-yl)propyl)amino)-4'-ethyl-[1,1'-biphenyl]-3-carboxylate;
methyl 4-(((2*S*)-2-((*tert*-butoxycarbonyl)amino)-1-cyano-3-(1*H*-indol-3-yl)propyl)amino)-4'-cyclopropyl-[1,1'-biphenyl]-3-carboxylate;
methyl 4-(((2*S*)-2-((*tert*-butoxycarbonyl)amino)-1-cyano-3-(1*H*-indol-3-yl)propyl)amino)-4'-(4-methylpiperazin-1-yl)-[1,1'-biphenyl]-3-carboxylate;
methyl 4-(((2*S*)-2-((*tert*-butoxycarbonyl)amino)-1-cyano-3-(1*H*-indol-3-yl)propyl)amino)-2'-fluoro-5'-isopropoxy-[1,1'-biphenyl]-3-carboxylate;
methyl 2-(((2*S*)-2-((*tert*-butoxycarbonyl)amino)-1-cyano-3-(1*H*-indol-3-yl)propyl)amino)-5-(6-(pyrrolidin-1-yl)pyridin-3-yl)benzoate;
methyl 2-(((2*S*)-2-((*tert*-butoxycarbonyl)amino)-1-cyano-3-(1*H*-indol-3-yl)propyl)amino)-5-(6-methylpyridin-3-yl)benzoate;
methyl 2-(((2*S*)-2-((*tert*-butoxycarbonyl)amino)-1-cyano-3-(1*H*-indol-3-yl)propyl)amino)-5-(6-morpholinopyridin-3-yl)benzoate;
methyl 2-(((2*S*)-2-((*tert*-butoxycarbonyl)amino)-1-cyano-3-(1*H*-indol-3-yl)propyl)amino)-5-(6-methoxypyridin-3-yl)benzoate;
methyl 2-(((2*S*)-2-((*tert*-butoxycarbonyl)amino)-1-cyano-3-(1*H*-indol-3-yl)propyl)amino) benzoate;
*tert*-Butyl 2-(((2*S*)-2-((*tert*-butoxycarbonyl)amino)-1-cyano-3-(1*H*-indol-3-yl)propyl)amino)-benzoate;
methyl 4-(((2*S*)-2-((*tert*-butoxycarbonyl)amino)-1-cyano-3-(1*H*-indol-3-yl)propyl)amino)-4'-butyl-[1,1'-biphenyl]-3-carboxylate;
methyl 4-(((2*S*)-2-((*tert*-butoxycarbonyl)amino)-1-cyano-3-(1*H*-indol-3-yl)propyl)amino)-4'-(diethylamino)-[1,1'-biphenyl]-3-carboxylate;
methyl 4-(((2*S*)-2-((*tert*-butoxycarbonyl)amino)-1-cyano-3-(1H-indol-3-yl)propyl)amino)-4'-(cyclopropyl)-[1,1'-biphenyl]-3-carboxylate;
methyl 4-(((2*S*)-2-((*tert*-butoxycarbonyl)amino)-1-cyano-3-(1*H*-indol-3-yl)propyl)amino)-4'-propoxy-[1,1'-biphenyl]-3-carboxylate;
methyl 4-(((2*S*)-2-((*tert*-butoxycarbonyl)amino)-1-cyano-3-(1*H*-indol-3-yl)propyl)amino)-3'-(*tert*-butyl)-[1,1'-biphenyl]-3-carboxylate;
methyl 4-(((2*S*)-2-((*tert*-butoxycarbonyl)amino)-1-cyano-3-(1*H*-indol-3-yl)propyl)amino)-3'-ethyl-[1,1'-biphenyl]-3-carboxylate;
methyl 4-(((2*S*)-2-((*tert*-butoxycarbonyl)amino)-1-cyano-3-(1*H*-indol-3-yl)propyl)amino)-3'-(pyrrolidin-1-yl)-[1,1'-biphenyl]-3-carboxylate;
methyl 4-(((2*S*)-2-((*tert*-butoxycarbonyl)amino)-1-cyano-3-(1*H*-indol-3-yl)propyl)amino)-3'-(dimethylamino)-[1,1'-biphenyl]-3-carboxylate;
methyl 4-(((2*S*)-2-((*tert*-butoxycarbonyl)amino)-1-cyano-3-(1*H*-indol-3-yl)propyl)amino)-3'-(trifluoromethoxy)-[1,1'-biphenyl]-3-carboxylate;
methyl 2-(((2*S*)-2-((*tert*-butoxycarbonyl)amino)-1-cyano-3-(1*H*-indol-3-yl)propyl)amino)-5-(6-(4-isopropylpiperazin-1-yl)pyridin-3-yl)benzoate;
methyl 2-(((2*S*)-2-((*tert*-butoxycarbonyl)amino)-1-cyano-3-(1*H*-indol-3-yl)propyl)amino)-5-(2-ethoxypyrimidin-5-yl)benzoate;
methyl 4-(((2*S*)-2-((*tert*-butoxycarbonyl)amino)-1-cyano-3-(1*H*-indol-3-yl)propyl)amino)-4'-butyl-[1,1'-biphenyl]-3-carboxylate;
methyl 3'-benzyl-4-(((2*S*)-2-((*tert*-butoxycarbonyl)amino)-1-cyano-3-(1*H*-indol-3-yl)propyl)-amino)-[1,1'-biphenyl]-3-carboxylate;
2-(((2S)-2-(*tert*-butoxycarbonylamino)-1-cyano-3-(1*H*-indol-3-yl)propyl)amino)-*N-*benzyl-benzamide;
(3*S*)-2-((2-bromophenyl)amino)-4-(1*H*-indol-3-yl)-3-(*tert*-butoxycarbonylamino) butanenitrile;
methyl 2-(((2*R*)-2-((*tert*-butoxycarbonyl)amino)-1-cyano-3-(1*H*-indol-3-yl)propyl)amino)-benzoate;
(3*S*)-3-(*tert*-butoxycarbonylamino)-2-((2-acetylphenyl)amino)-4-(1*H*-indol-3-yl)butanenitrile;and
methyl 4-(((2*S*)-2-((*tert*-butoxycarbonyl)amino)-1-cyano-3-(1*H*-indol-3-yl)propyl)amino)-benzoate. As further detailed in the Examples below, these compounds are particularly active in the prevention or treatment of infection with coronavirus.

The compound of formula (I) of the first aspect of the invention may also be in the form of a pharmaceutically acceptable salt. Suitable anions forming pharmaceutically acceptable salts are known in the art and include, for instance, aspartate, benzenesulfonate, benzoate, besylate, bicarbonate, bitartrate, bromide, camsylate, carbonate, chloride, citrate, decanoate, edetate, esylate, fumarate, gluceptate, gluconate, glutamate, glycolate, hexanoate, hydroxynaphthoate, iodide, isethionate, lactate lactobionate, malate, maleate, mandelate, mesylate, methylsulfate, mucate, napsylate, nitrate, octanoate, oleate, pamoate, pantothenate, phosphate, polygalacturonate, propionate, salicylate, stearate, acetate, succinate, sulfate, tartrate, teoclate, and tosylate.

Suitable cations forming pharmaceutically acceptable salts are known in the art and include, for instance, aluminium, argininium, calcium, lithium, potassium, sodium and zinc.

As detailed above, the second aspect of the invention relates to a composition comprising a compound of formula (I) or a compound of formula (11) or a compound of formula (12) as defined in any of the particular and preferred embodiments of the first aspect of the invention defined above, or a pharmaceutically acceptable salt thereof and one or more pharmaceutically acceptable diluent or carrier.

The composition according to the present invention may be in any form suitable for the application to humans and/or animals, preferably humans including infants, children and adults, and can be produced by standard procedures known to those skilled in the art. Therefore, the composition in accordance with the invention may be adapted for topical or systemic application, particularly for dermal, transdermal, subcutaneous, intramuscular, intra-articular, intraperitoneal, intravenous, intra-arterial, intravesical, intraosseous, intracavernosal, pulmonary, buccal, sublingual, ocular, intravitreal, intranasal, percutaneous, rectal, vaginal, oral, epidural, intrathecal, intraventricular, intracerebral, intracerebroventricular, intracisternal, intraspinal, perispinal, intracranial, delivery via needles or catheters with or without pump devices, or other application routes, such as spray injection or infusion.

The composition according to the second aspect of the invention may further be transferred into usual formulations, such as tablets, sugar-coated tablets, pills, granules, aerosols, syrups, emulsions, suspensions, solutions, ointments, creams, dermal patches and gel of any kind, using in particular essentially non-toxic and pharmaceutically acceptable carriers and diluents.

The composition of the second aspect of the invention may in certain embodiments further comprise auxiliary materials or additives of a pharmaceutical composition selected among excipients, support materials, lubricants, fillers, solvents, colorants, flavour conditioners such as sugars, antioxidants, binders, adhesives, disintegrants, anti-adherents, glidants and/or agglutinants. In the case of suppositories, this may imply waxes or fatty acid esters or preservatives, emulsifiers and/or carriers for parenteral application. The selection of these auxiliary materials and/or additives and the amounts to be used will depend on the form of application of the pharmaceutical composition and will become apparent to the skilled person.

In an embodiment the pharmaceutical compositions of the second aspect of the invention are a form suitable for oral administration, either solid or liquid. Suitable dose forms for oral administration may be tablets, pills, caplets, gel caps, chewing gums, capsules, granules, drops, syrups or solutions and may contain conventional excipients known in the art such as binding agents, for example syrup, acacia, gelatin, sorbitol, tragacanth, or polyvinylpyrrolidone; fillers, for example lactose, sugar, maize starch, calcium phosphate, sorbitol or glycine; tabletting lubricants, for example magnesium stearate; disintegrants, for example starch, polyvinylpyrrolidone, sodium starch glycollate or microcrystalline cellulose; or pharmaceutically acceptable wetting agents such as sodium lauryl sulfate.

The solid oral compositions may be prepared by conventional methods of blending, filling or tabletting. Repeated blending operations may be used to distribute the active agent throughout those compositions employing large quantities of fillers. Such operations are conventional in the art. The tablets may for example be prepared by wet or dry granulation and optionally coated according to methods well known in normal pharmaceutical practice, in particular with an enteric coating.

The composition of the second aspect may also be adapted for parenteral administration, such as sterile solutions, suspensions or reconstitutable dry preparations, aerosols or sprays in the appropriate unit dosage form. Adequate excipients can be used, such as bulking agents, buffering agents or surfactants.

In a preferred embodiment of the second aspect, the composition comprises a therapeutically effective amount of the compound of formula (I) as defined in the first aspect of the invention. The physician will determine the dosage of the present therapeutic agents which will be most suitable and it will vary with the form of administration and the particular compound chosen, and furthermore, it will vary with the patient under treatment, the age of the patient, the type of disease or condition being treated. For instance, when the composition is administered orally, larger quantities of the active agent will be required to produce the same effect as a smaller quantity given parenterally. The compounds are useful in the same manner as comparable anti-infective agents and the dosage level is of the same order of magnitude as is generally employed with these other anti-infective agents.

The compounds and compositions of this invention may be used with other drugs to provide a combination therapy. The other drugs may form part of the same composition, or be provided as a separate composition for administration at the same time or at different time. The compound of formula (I) may thus be combined with other anti-viral agents, such as remdesivir, nitazoxanide, nirmatrelvir, ivermectin and molnupiravir, as well as with antibodies having anti-viral activity.

As defined above, the inventors have found that the compounds of formula (I) are particularly useful in the prevention or treatment of infection with coronavirus.

A third aspect of the invention thus relates to a compound of formula (I) or a compound of formula (11) or a compound of formula (12) as defined in the first aspect of the invention, or a pharmaceutically acceptable salt thereof, or a composition as defined in the second aspect of the invention for use in medicine.

A fourth aspect of the invention relates to a compound of formula (I) or a compound of formula (11) or a compound of formula (12) as defined in the first aspect of the invention, or a pharmaceutically acceptable salt thereof, or a composition as defined in the second aspect of the invention for use in in the treatment or prevention of viral infection by coronavirus.

The fourth aspect of the invention also relates to a method of prevention or treatment of infection with a coronavirus, said method comprising the step of administering a therapeutically active amount of a compound of formula (I) or of a compound of formula (11) or of a compound of formula (12) as defined in the first aspect of the invention, or of a pharmaceutically acceptable salt thereof, or of a composition as defined in the second aspect of the invention to a patient susceptible of being or being infected with coronavirus.

The fourth aspect of the invention also relates to the use of a compound of formula (I) or of a compound of formula (11) or of a compound of formula (12) as defined in the first aspect of the invention, or of a pharmaceutically acceptable salt thereof, or of a composition as defined in the second aspect of the invention in the prevention or treatment of infection with coronavirus.

A process for the preparation of a compound of formula (I) is also part of the invention.

Thus, the fifth aspect of the invention relates to a process for the preparation of a compound of formula (I) as defined in any of claims 1 to 9 comprising the steps of:
(a) contacting a compound of formula (II) with a compound of formula (III) 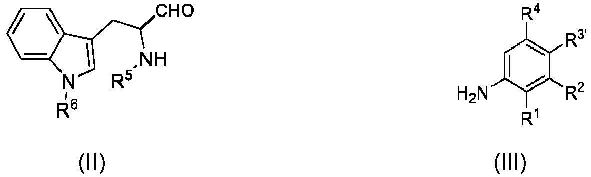 in order to form a compound of formula (IV) 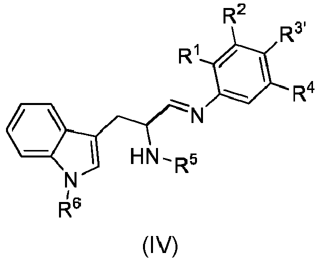 wherein R¹, R², R⁴, R⁵ and R⁶ are as defined in any of the particular and preferred embodiments of the first aspect of the invention,
   R^{3'} receives the same definition as the R³ group in any of the particular and preferred embodiments of the first aspect of the invention,
and (b) treating the product of step (a) with a cyanide source in order to form a product of formula (I).

In a preferred embodiment of the fifth aspect of the invention, the cyanide source of step (b) is selected from the group consisting of trimethylsilyl cyanide and potassium cyanide. Trimethylsilyl cyanide is a preferred cyanide source, because it is soluble in organic solvents such as methanol and ethanol.

In a preferred embodiment of the fifth aspect of the invention, steps (a) and (b) are carried out without isolating the compound of formula (IV).

In a preferred embodiment of the fifth aspect of the invention, step (a) is carried out by heating a solution of the compounds of formulae (II) and (III) in the presence of a Lewis acid, such as zinc(II) chloride. A suitable temperature may be between 50 °C and 100 °C. Alternative conditions for the formation of an imine functional group by condensation of an amine with an aldehyde, are known in the art and will become apparent to the skilled person.

In a preferred embodiment of the fifth aspect of the invention, step (b) is carried out at room temperature.

In a preferred embodiment of the fifth aspect of the invention, each of steps (a) and (b) are carried out in methanol.

In a preferred embodiment, the process of the fifth aspect of the invention comprises the previous step of preparing the compound of formula (II) by treating a compound of formula (V) with a hydride source

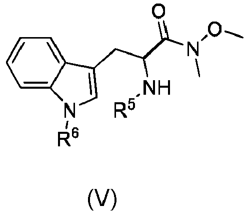

The hydride source of said previous step is preferably lithium aluminium hydride.

In a preferred embodiment, the process of the fifth aspect of the invention comprises the previous step of preparing the compound of formula (V) by contacting a compound of formula (VI) with dimethylhydroxylamine in the presence of a peptidic coupling agent.

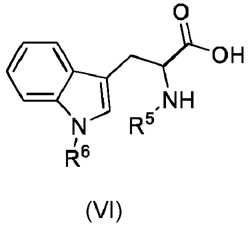

Suitable peptidic coupling agents are well known in the art and will become apparent to the skilled person. For instance, a suitable peptidic agent may be *N,N'-*diisopropylcarbodiimide combined with a base, such as 4-dimethylaminopyridine (DMAP).

In a particular embodiment of the fifth aspect of the invention, the process of the fifth aspect of the invention comprises the steps of:
(a) contacting a compound of formula (II) with a compound of formula (IIIa) 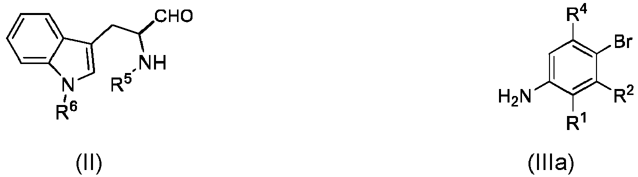 in order to form a compound of formula (IVa) 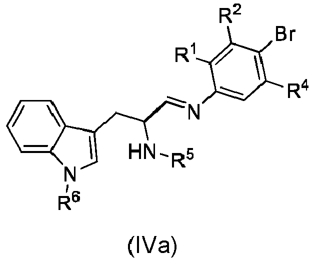 wherein R¹, R², R⁴, R⁵ and R⁶ are as defined in any of the particular and preferred embodiments of the first aspect of the invention,
(b) treating the product of step (a) with a cyanide source in order to form a product of formula (V) 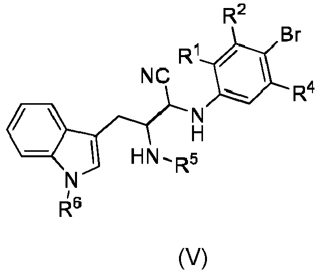
and (c) the step of contacting the compound of formula (V) with a compound of formula R³-B(OH)₂ in the presence of a catalytically effective amount of a cross-coupling catalyst, wherein R³ is a group selected from the group consisting of pyridinyl, pyrimidinyl and phenyl, said group being optionally substituted at any available position with one or two groups selected from the group consisting of halogen, (C₁-C₄)alkyl, (C₁-C₄)alkyloxy, trilfuoromethyloxy, di((C₁-C₄)alkyl)amino, phenyl, benzyl, (C₃-C₆)cycloalkyl and a 5 to 6-membered saturated heterocyclic ring, the members of the ring being selected from the group consisting of C, CH, CH₂, O, N and NH, said ring being optionally substituted at any available position with a (C₁-C₄)alkyl; or, alternatively, wherein said group selected from the group consisting of pyridinyl, pyrimidinyl and phenyl optionally forms a 6-membered ring with a divalent group of formula -O-(CH₂)₂-O- whereby the oxygen atoms of said divalent group are connected to two adjacent carbon atoms of said pyridinyl, pyrimidinyl or phenyl group.

Said further step of cross-coupling is preferably carried out in the presence of a base. Suitable bases for cross-coupling reactions, such as Suzuki couplings, are well known in the art and will become apparent to the skilled person. It is preferred that the base is an alkaline carbonate salt, such as potassium carbonate.

Suitable catalysts for cross-coupling reactions, such as Suzuki couplings, are well known in the art and will become apparent to the skilled person. It is preferred that said catalyst is a palladium(0) catalyst, such as tetrakis(triphenylphosphine)palladium.

Throughout the description and claims the word "comprises" and variations of the word, are not intended to exclude other technical features, additives, components or steps. Furthermore, the word "comprise" encompasses the cases of "consist of" and "consists essentially of". Additional objects, advantages and features of the invention will become apparent to those skilled in the art upon examination of the description or may be learned by practice of the invention. The following examples are provided by way of illustration, and they are not intended to be limiting of the present invention.

### EXAMPLES

### General considerations

Reactions were monitored either by TLC and/or analytic HPLC (UV, detection, 220 nm). Flash columns, filled with silica gel Merck 60 (230-400) were used for chromatographic separations. A reversed-phase column, Sunfire C18 (4.6 × 50 mm, 3.5 µm), a flux of 1 mL/min, and mixtures of CH₃CN, phase A, and H₂O, phase B, both containing 0.01% formic acid, were used for analytical HPLCs. Mass spectra, in electrospray, positive mode, were obtained on a Waters Micromass ZQ spectrometer. The peaks corresponding to the molecular ion have been indicated as ([M]⁺), while those corresponding to the molecular ion plus one proton as ([M+H]⁺). NMR spectra were recorded in a Varian INOVA-400 (400 MHz) spectrometer, operating at 400 and 100 MHz for ¹H and ¹³C experiments, respectively (with chemical shifts expressed in ppm and coupling constants in Hz).

### SYNTHESIS OF COMPOUNDS OF FORMULA (I)

### Preparative Example 1: Preparation of tert-butyl (S)-(3-(1H-indol-3-yl)-1-(methoxy(methyl)amino)-1-oxopropan-2-yl)carbamate

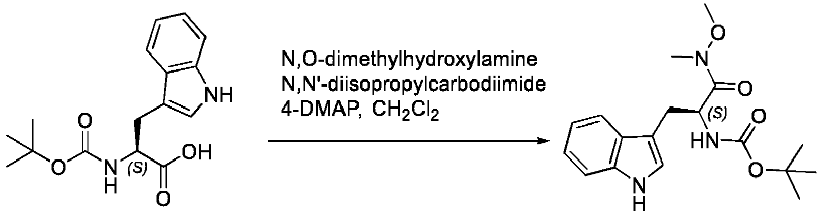

To a slurry of *N*-(tert-butoxycarbonyl)-L-tryptophan (1 mmol) and *N*,*O-*dimethylhydroxylamine hydrochloride (1 mmol) in dry dichloromethane (7.5 mL) under argon atmosphere, were added 4-dimethylaminopyridine (1.1 mmol) and *N*,*N*'-diisopropylcarbodiimide (1.1 mmol). After stirring for 12 hours at room temperature, a solution of HCl 1N (3 mL) was added and the organic layer washed with water (3 mL) and brine (3 mL). The organic phase was dried over anhydrous Na₂SO₄ and evaporated to dryness. The crude mixture was purified by flash column chromatography on silica gel using 0% to 40% of EtOAc in hexane as eluant to obtain the title compound in a 93% yield.

### Preparative Example 2: Preparation of tert-butyl (S)-(1-(1H-indol-3-yl)-3-oxopropan-2-yl)carbamate (IIa)

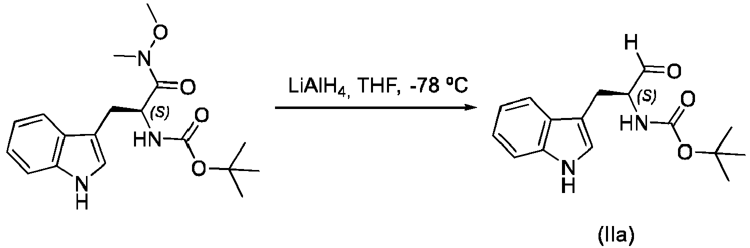

A solution of the product prepared in Preparative Example 1 (1 mmol) in anhydrous THF (7 mL) was added dropwise to a suspension of LiAlH₄ (2 mmol) in anhydrous THF (10 mL) previously cooled down to -78 °C. The reaction mixture was kept at -78 °C for 1h. After this time, a solution of KHSO₄ (1.6 mmol, 0.4 M in water, 17 mL) and Et₂O (17 mL) were added, and the reaction mixture was allowed to reach room temperature. The resulting phases were separated, and the aqueous phase was additionally extracted with Et₂O (2 × 9 mL). The combined organic phases were washed with citric acid 10% (2 × 14 mL), water (2 × 14 mL), NaHCO₃ sat. (2 × 14 mL), brine (2 × 14 mL), and dried over anhydrous Na₂SO₄. After filtration and evaporation to dryness, the desired product was obtained in a 85% yield and used without further purification.

### Example 1: Preparation of compounds of formula (I)

Two synthetic procedures were adopted for the synthesis of compounds of formula (I):
General Method 1: 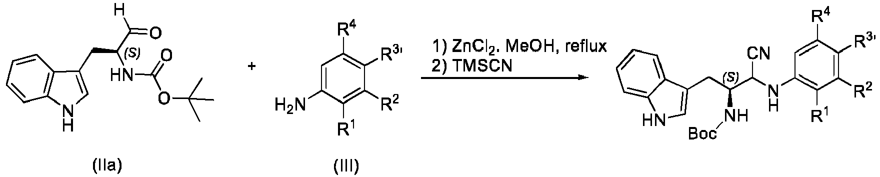
General Method 2:

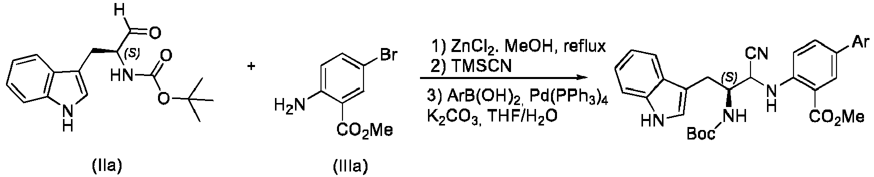

General Procedure for General Method 1: The corresponding amine compound of formula (III) (2 mmol) and ZnCl₂ (1.1 mmol) were added to a solution of the compound of formula (IIa) (1 mmol) in MeOH (20 mL), and the mixture was refluxed for 12 h. After this time, the reaction mixture was allowed to cool at room temperature, and trimethylsilyl cyanide (TMSCN) (1.09 mmol) was added. After stirring for 24 h at room temperature, the solvent was evaporated and the residue was dissolved in EtOAc (20 mL). This solution was successively washed with H₂O (2x5 mL), brine (5 mL), dried over Na₂SO₄ and evaporated to dryness. The crude mixture was purified by flash chromatography using the eluent system indicated in each case.

General Procedure for General Method 2: The amine compound of formula (IIIa) (2 mmol) and ZnCl₂ (1.1 mmol) were added to a solution of the compound of formula (IIa) (1 mmol) in MeOH (20 mL), and the mixture was refluxed for 12 h. After this time, the reaction mixture was allowed to cool at room temperature, and TMSCN (1.09 mmol) was added. After stirring for 24 h at room temperature, the solvent was evaporated and the residue was dissolved in EtOAc (20 mL). This solution was successively washed with H₂O (2x5 mL), brine (5 mL), dried over Na₂SO₄ and evaporated to dryness. The crude mixture was purified by flash chromatography using 0% to 40% of EtOAc in hexane gradient as eluant, which furnished the desired compound of formula (Ia) as an epimeric mixture in a ratio 1:3/*1R*,*2S*:*1S*,*2S* in 22% yield.

The product obtained in the previous step (1.0 equiv), the boronic acid of formula R³B(OH)₂ wherein R³ is as defined in Table 1 (1.5 equiv) and K₂CO₃ (6 equiv) were dissolved in THF/H₂O (5 mL/mmol , 4/1) in a microwave vial. Argon was bubbled for 10 min., then [Pd(PPh₃)₄] (2%) was added under a positive pressure of Argon. The vial was sealed, evacuated and backfilled with Argon, and then irradiated in a microwave apparatus at 100 °C, for 30 min. After the reaction mixture was cooled to room temperature, the solvent was evaporated to dryness. The resulting solid was suspended in water (10 mL) and the aqueous phase was extracted with EtOAc (3x10 mL). The combining organic phases were dried over Na₂SO₄, filtrated and evaporated to dryness. The crude mixture was purified by flash chromatography using the eluent system indicated in each case.

Table 1 below summarizes the results obtained for the preparation of a compound of formula (I) according to general methods 1 or 2, wherein R⁴ is hydrogen unless otherwise indicated, R⁵ is *tert*-butoxycarbonyl, R⁶ is hydrogen and wherein R¹, R², R³ and R⁴ are as defined Table 1:

**Table 1**

| Product | Method | R¹ | R² | R³ | Eluant | Yield (%) | d.r. |
|---|---|---|---|---|---|---|---|
| (Ia) | 2 | CO₂Me | H | 4-N-(morpholinyl)phenyl | 10% to 50% | 76 | 1:2 |
| | | | | | AcOEt in Hexane | | |
| (Ib) | 1 | CO₂Me | H | Br | 0% to 40% AcOEt in Hexane | 22 | 1:3 |
| (Ic) | 1 | CO-Ph | H | Br | | 55 | 1:2 |
| (Id) | 1 | CO-Ph | H | Cl | 0% to 20% AcOEt in Hexane | 57 | 1:2 |
| (Ie) | 1 | CO₂Me | H | Br | 0% to 40% AcOEt in Hexane | 60 | 1:2 |
| (If) | 2 | CO₂Me | H | 2,3-dihydro-benzo[1,4]dioxin-6-yl | | 57 | 1:1.5 |
| (Ig) | 2 | CO₂Me | H | 3-N-(morpholinyl)phenyl | 0% to 50% AcOEt in Hexane | 60 | 1:1 |
| (Ih) | 1 | CO₂Et | H | H | 5% to 30% AcOEt in Hexane | 64 | 1:2 |
| (Ii) (R⁴=Br) | 1 | CO₂Me | H | H | 20-100% MeCN in water | 8 | 1:2 |
| (Ij) | 2 | CO₂Me | H | 4-*tert*-butylphenyl | 5% to 30% AcOEt in Hexane | 25 | 1:2 |
| (Ik) | 1 | CO₂Me | Br | H | | 65 | 1:2 |
| (Ii) | 2 | CO₂Me | H | 4-ethylphenyl | 0% to 30% AcOEt in Hexane | 78 | 1:2 |
| (Im) | 2 | CO₂Me | H | 4-cyclopropylphenyl | 5% to 30% AcOEt in Hexane | 60 | 1:2(Im1) |
| | | | | | | | 1:5(Im2) |
| | | | | | | | 1:3(Im3) |
| | | | | | | | 2:1(Im4) |
| (In) | 2 | CO₂Me | H | 4-(4-methylpiperazin-1-yl)phenyl | 0% to 10% MeOH in DCM | 60 | 1:1.5 |
| (Io) | 2 | CO₂Me | H | 4-N-(morpholinyl)phenyl | 0% to 30% AcOEt in Hexane | 53 | 1:3 |
| (Ip) | 2 | CO₂Me | H | 2-fluoro-5-isopropyloxyphenyl | 0% to 40% AcOEt in Hexane | 30 | 1:1.3 |
| (lq) | 2 | CO₂Me | H | 6-pyrrolidin-pyrid in-3-yl | 20% to 50% AcOEt in Hexane | 41 | 1:1 |
| (Ir) | 2 | CO₂Me | H | 6-methyl-pyridin-3-yl | 0% to 60% AcOEt in Hexane | 54 | 1:1.5 |
| (Is) | 2 | CO₂Me | H | 6-(N-morpholinyl)pyridine-3-yl | 0% to 50% AcOEt in Hexane | 53 | 1:2 |
| (It) | 2 | CO₂Me | H | 6-methoxy-pyridin-3-yl | 0% to 40% AcOEt in Hexane | 60 | 1:1.3 |
| (Iu) | 1 | CO₂Me | H | H | 5% to 30% AcOEt in Hexane | 37 | 1:3 |
| (Iv) | 1 | CO₂^{t}But | H | H | | 15 | 1:2 |
| (Iw) | 2 | CO₂Me | H | 4-butylphenyl | 0% to 30% AcOEt in Hexane | 41 | 1:2 |
| (Ix) | 2 | CO₂Me | H | 4-diethylaminophenyl | 5% to 30% AcOEt in Hexane | 10.6 | 1:2 |
| (Iy) | 2 | CO₂Me | H | 4-propoxyphenyl | | 50 | 1:1.5 |
| (Iz) | 2 | CO₂Me | H | 3-*tert*-butylphenyl | 0% to 30% AcOEt in Hexane | 50 | 1:2 |
| (Iaa) | 2 | CO₂Me | H | 3-ethylphenyl | | 52 | 1:1.5 |
| (lab) | 2 | CO₂Me | H | 3-(*N-*pyrrolidinyl)phenyl | | 83 | 1:1.5 |
| (lac) | 2 | CO₂Me | H | 3-diethylaminophenyl | | 56 | 1:1.5 |
| (lad) | 2 | CO₂Me | H | 3-trifluoromethyloxy-phenyl | | 52 | 1:2 |
| (lae) | 2 | CO₂Me | H | 6-(4-isopropylpiperazin-1-yl)pyridin-3-yl | 0% to 10% MeOH in DCM | 38 | 1:1 |
| (Iaf) | 2 | CO₂Me | H | 2-ethoxy-pyrimidin-5-yl | 0% to 50% AcOEt in Hexane | 15 | 1:2 |
| (lag) | 2 | CO₂Me | H | 4-butylphenyl | 5% to 30% AcOEt in Hexane | 53 | 1:1.5 |
| (lah) | 2 | CO₂Me | H | 3-benzylphenyl | 0% to 40% AcOEt in Hexane | 54 | 1:1.5 |
| (lai) | 1 | CO-NH-CH₂-Ph | H | H | 0% to 50% AcOEt in Hexane | 41 | 1:3 |
| (Iaj) | 1 | Br | H | H | | 73 | 1:2 |
| (Iak) | 1 | CO₂Me | H | H | 5% to 30% AcOEt in Hexane | 57 | 1:3 |
| (lal) | 1 | CO-Me | H | H | | 12 | 1:3 |
| (lam) | 1 | H | H | CO₂Me | 5% to 30% AcOEt in Hexane | 69 | 1:2 |

Diastereomeric ratios of Table 1 are expressed as the molar ratio of the diastereomer of configuration (R,S) to the diastereomer of configuration (S,S), whereby the diastereomer of configuration (R,S) is that wherein the carbon atom bearing the CN group is in the configuration (R) and the carbon atom bearing the NHBoc group is in the configuration (S); and whereby, similarly, the diastereomer of configuration (S,S) is that wherein the carbon atom bearing the CN group is in the configuration (S) and the carbon atom bearing the NHBoc group is in the configuration (S).

Table 2 below provides characterization information (¹H NMR, ¹³C NMR and LC-MS) for the compounds of formula (I) of Table 1:

**Table 2**

| Product | Characterization information |
|---|---|
| (Ia) | ¹H-NMR (400 MHz, CDCl₃) δ (ppm): 8.32 (t, *J* = 7.8 Hz, 1.77H), 8.24-8.13 (m, 3.5H), 7.71 (d, *J* = 8.2 Hz, 1H), 7.65 (dd, *J* = 8.7, 2.4 Hz, 1H), 7.59 (d, *J* = 7.9 Hz, 1H), 7.54 (d, *J* = 8.6 Hz, 1.5H), 7.46 (dd, *J* = 13.8, 8.7 Hz, 4H), 7.38 (t, *J* = 7.8 Hz, 2.5H), 7.22 (qd, J = 7.7, 1.2 Hz, 2.6H), 7.18-7.07 (m, 5H), 6.98 (t, *J* = 8.4 Hz, 4H), 6.91 (s, 1.4H), 6.57 (d, *J =* 8.9 Hz, 1H), 4.95 (d, *J* = 9.5 Hz, 1H), 4.84 (s, 1.5H), 4.66 (s, 1.5H), 4.54-4.40 (m, 2H), 3.91 (s, 5.3H), 3.88 (s, 7H), 3.36 (ddd, J = 26.2, 14.3, 5.2 Hz, 2H), 3.20 (q, *J* = 5.7 Hz, 7H), 1.64 (s, 2H), 1.51 (s, 9H), 1.42 (s, 7H), 1.26 (s, 1H). ¹³C-NMR (100 MHz, CDCl₃) δ (ppm): 168.97, 168.67, 155.53, 150.34, 147.01, 146.62, 136.50, 133.19, 132.82, 130.32, 129.69, 129.62, 127.23, 123.14, 122.74, 122.64, 120.19, 120.09, 118.96, 118.80, 117.97, 117.60, 116.10, 112.83, 112.64, 112.56, 111.48, 111.42, 110.26, 110.12, 80.76, 77.36, 67.00, 52.64, 52.09, 51.95, 49.48, 48.61, 48.49, 28.61, 28.47, 28.38. LC-MS (m/z): 610.37 ([M+H]⁺) |
| (Ib) | ¹H-NMR (400 MHz, CDCl₃) δ (ppm): 8.20 (d, *J* = 18.2 Hz, 1.33H), 8.10 (d, *J* = 15.7 Hz, 1.33H), 7.93 (d, *J* = 12.6 Hz, 1.33H), 7.56 (d, *J* = 7.7 Hz, 1H), 7.44 (d, *J* = 7.9 Hz, 0.33H), 7.36 (dd, J = 8.9, 2.4 Hz, 0.33H), 7.26 (t, *J* = 7.5 Hz, 2.33H), 7.16-7.07 (m, 2H), 7.02 (q, *J* = 6.4, 5.0 Hz, 2.33H), 6.96 (d, *J* = 11.6 Hz, 0.33H), 6.24 (d, *J* = 8.8 Hz, 1H), 4.83 (d, *J* = 9.7 Hz, 1H), 4.73 (dd, J = 5.7, 2.9 Hz, 0.33H), 4.66-4.58 (m, 0.33H), 4.58-4.46 (m, 1H), 4.45-4.38 (m, 0.33H), 4.33 (ddd, J = 13.5, 8.0, 5.7 Hz, 0.33H), 4.24 (d, *J* = 2.3 Hz, 1H), 3.76 (d, *J* = 2.8 Hz, 1H), 3.73 (s, 3H), 3.21 (ddd, J = 18.3, 15.1, 5.4 Hz, 1.33H), 3.05 (dd, J = 14.6, 8.9 Hz, 1.66H), 1.39 (s, 9H), 1.29 (s, 3H), 1.20 (s, 1.33H). ¹³C-NMR (100 MHz, CDCl₃) δ (ppm): 167.79, 167.52, 155.59, 147.16, 146.76, 137.29, 136.47, 134.29, 127.06, 123.16, 123.15, 122.75, 122.64, 120.17, 120.07, 120.07, 118.85, 118.84, 117.59, 117.16, 113.88, 113.75, 111.54, 111.53, 109.95, 109.17, 100.01, 81.00, 52.43, 52.29, 52.15, 48.32, 28.71, 28.70, 28.42, 1.15. LC-MS (m/z): 549.13 ([M+Na]⁺). |
| (Ic) | ¹H-NMR (400 MHz, CDCl₃) δ (ppm): 8.76 (t, *J* = 8.8 Hz, 1.5H), 8.26 (s, 1H), 8.21 (s, 0.5H), 7.69 (d, *J* = 7.8 Hz, 1.5H), 7.58 (dd, J = 22.2, 7.1 Hz, 8.5H), 7.52-7.43 (m, 4.5H), 7.39 (d, *J* = 8.1 Hz, 2.5H), 7.34 (d, *J* = 8.1 Hz, 1H), 7.26-7.18 (m, 2H), 7.18-7.12 (m, 2.5H), 7.12-7.05 (m, 1H), 6.86 (d, *J* = 9.1 Hz, 0.5H), 6.65 (d, *J* = 8.8 Hz, 0.5H), 6.47 (d, *J* = 8.8 Hz, 1H), 4.94 (d, *J* = 9.4 Hz, 1H), 4.83 (d, *J* = 23.4 Hz, 1H), 4.68-4.58 (m, 1H), 4.50-4.41 (m, 2H), 3.49 (s, 0.5H), 3.35 (ddd, J = 23.4, 15.0, 5.8 Hz, 2H), 3.19 (dd, *J* = 14.7, 8.6 Hz, 2H), 1.69-1.59 (m, 1H), 1.46 (s, 9H), 1.40 (s, 4.5H), 1.26 (t, *J* = 7.2 Hz, 1H). ¹³C-NMR (100 MHz, CDCl₃) δ (ppm): 209.51, 206.56, 197.92, 155.54, 155.52, 155.34, 147.60, 147.18, 138.94, 137.36, 137.35, 137.14, 136.99, 136.52, 136.34, 132.04, 131.95, 131.68, 129.51, 129.49, 129.43, 129.25, 128.54, 128.44, 127.12, 123.16, 122.83, 122.69, 121.14, 120.24, 120.11, 118.94, 118.70, 117.45, 117.05, 114.29, 114.17, 111.52, 111.47, 111.26, 110.12, 108.55, 80.92, 77.36, 52.44, 48.41, 48.32, 48.31, 48.31, 31.09, 28.54, 28.50, 28.41, 28.36. LC-MS (m/z): 597.24 ([M+Na]⁺). |
| (Id) | ¹H-NMR (400 MHz, CDCl₃) δ (ppm): 8.75 (t, *J* =8.1 Hz, 1.5H), 8.23 (s, 1H), 8.19 (s, 0.5H) 7.70 (d, J = 8.4 Hz, 1H) 7.61 (dd, J = 8.3, 1.5 Hz, 3H) 7.59-7.54 (m, 2H) 7.52-7.44 (m, 4.5H) 7.35 (d, *J* = 8.2 Hz, 0.5H) 7.26-7.19 (m, 1.5H) 7.18-7.12 (m, 2.5H) 7.11-7.06 (m, 1H) 6.91 (d, *J* = 8.9 Hz, 0.5H) 6.53 (d, *J* = 9.0 Hz, 1H) 4.93 (d, *J* = 9.2 Hz, 1H), 4.83 (d, *J =* 14.3 Hz, 1H) 4.68-4.59 (m, 1H) 4.50-4.42 (m, 1.5H) 3.35 (ddd, *J* = 24.6, 15.3, 6.2 Hz, 1.5H) 3.19 (dd, *J* = 14.8, 8.6 Hz, 1.5H) 2.02-1.79 (m, 1H) 1.45 (s, 9H) 1.40 (s, 4.5H) ¹³C-NMR (100 MHz, , CDCl₃) δ (ppm): 198.51, 198.01, 155.55, 147.22, 146.79, 138.97, 136.54, 135.10, 134.59, 134.31, 134.24, 132.02, 131.93, 129.48, 129.40, 128.52, 128.43, 127.13, 123.19, 122.77, 122.64, 121.72, 120.63, 120.19, 120.06, 118.90, 118.66, 117.52, 117.13, 113.94, 113.82, 111.55, 111.49, 110.06, 109.87, 52.49, 52.11, 48.48, 28.38, 26.39. LC-MS (m/z): 551.22 ([M+Na]+). |
| (Ie) | ¹H-NMR (400 MHz, CDCl₃) δ (ppm): 8.34 (d, *J* = 8.3 Hz, 1.11H), 8.29 (d, *J* = 7.7 Hz, 0.43H), 8.16 (s, 1.03H), 8.13 (s, 0.5H), 8.06 (d, *J* = 2.4 Hz, 0.56H), 8.04 (d, *J* = 2.4 Hz, 1.02H), 7.69 (d, *J* = 8.1 Hz, 1H), 7.56 (d, *J* = 7.9 Hz, 0.54H), 7.50 (dd, J = 8.9, 2.6 Hz, 0.54H), 7.39 (t, *J* = 7.9 Hz, 2.77H), 7.23 (d, *J* = 8.1 Hz, 1.53H), 7.18-7.07 (m, 3.42H), 6.76 (d, 0.43H), 6.37 (d, *J* = 8.9 Hz, 1H), 4.90 (d, *J* = 9.7 Hz, 1H), 4.77 (d, *J =* 12.5 Hz, 1H), 4.64 (m, 1H), 4.47-4.39 (m, 0.63H), 4.39-4.32 (m, 1.25H), 3.88 (s, 1.25H), 3.85 (s, 3.11H), 3.39-3.27 (m, 1H), 3.19 (d, *J* = 8.8 Hz, 1.1H), 3.15 (d, *J* = 8.9 Hz, 0.66H), 1.50 (s, 9H), 1.41 (s, 4.5H), 1.20 (m, 0.63H). ¹³C-NMR (100 MHz, CDCl₃) δ (ppm): 167.80, 167.53, 155.51, 147.23, 146.84, 137.70, 137.32, 136.52, 134.33, 127.12, 123.13, 122.81, 122.70, 120.23, 120.12, 118.90, 118.69, 117.59, 117.17, 113.96, 113.79, 111.54, 110.11, 109.93, 109.19, 80.89, 52.48, 52.29, 52.15, 48.47, 48.38, 28.74, 28.44, 28.37. LC-MS (m/z): 549.23 ([M+Na]⁺). |
| (If) | ¹H-NMR (400 MHz, CDCl₃) δ (ppm): 8.33 (t, *J* = 8.7 Hz, 1.66H), 8.15 (d, *J* = 2.3 Hz, 1.66H), 8.12 (d, *J* = 2.4 Hz, 1.66H), 7.71 (d, *J* = 7.7 Hz, 1H), 7.64-7.57 (m, 1.66H), 7.51 (d, *J* = 7.9 Hz, 1H), 7.39 (t, *J* = 7.9 Hz, 1.66H), 7.26-7.19 (m, 2H), 7.17 (s, 1.32H), 7.14 (d, J= 8.4 Hz, 1.32H), 7.12-7.08 (m, 1H), 7.07 (d, *J* = 2.1 Hz, 1H), 7.05-7.00 (m, 2.32H), 6.99 (d, *J* = 2.2 Hz, 0.66H), 6.90 (t, *J* = 8.4 Hz, 2.32H), 6.56 (d, *J* = 8.8 Hz, 1H), 4.92 (d, *J* = 9.4 Hz, 1H), 4.81 (s, 1H), 4.66 (s, 1H), 4.53-4.42 (m, 2H), 4.30 (s, 2.66H), 4.28 (s, 4H), 3.90 (s, 2H), 3.87 (s, 3H), 3.43-3.28 (m, 2H), 3.22 (d, *J* = 8.4 Hz, 0.66H), 3.19 (d, *J* = 8.3 Hz, 1H), 1.51 (s, 9H), 1.41 (s, 6H). ¹³C-NMR (100 MHz, CDCl₃) δ (ppm): 168.93, 168.64, 155.51, 147.23, 146.84, 143.87, 143.83, 142.95, 142.91, 136.50, 133.79, 133.31, 132.93, 130.12, 130.04, 129.96, 129.90, 127.42, 127.24, 123.12, 122.78, 122.67, 120.22, 120.12, 119.57, 118.99, 118.81, 117.95, 117.74, 117.69, 117.56, 115.24, 112.77, 112.58, 112.49, 111.48, 111.41, 110.28, 110.15, 80.77, 77.36, 64.61, 52.62, 52.09, 51.95, 48.57, 48.45, 28.62, 28.47, 28.38. LC-MS (m/z): 605.29 ([M+Na]⁺). |
| (Ig) | ¹H-NMR (400 MHz, CDCl₃) δ (ppm): 8.37 (t, *J* = 8.1 Hz, 2H), 8.20-8.11 (m, 4H), 7.71 (d, *J* = 7.6 Hz, 1H), 7.67 (dd, *J* = 8.6, 2.4 Hz, 1H), 7.61-7.53 (m, 2H), 7.39 (t, *J* = 7.9 Hz, 2H), 7.32 (q, *J* = 8.3 Hz, 2H), 7.25-7.19 (m, 2H), 7.17 (d, *J* = 7.9 Hz, 2H), 7.15-7.09 (m, 2H), 7.05 (dd, *J* = 14.5, 6.8 Hz, 4H), 6.93 (d, *J* = 7.8 Hz, 1H), 6.88 (dt, *J* = 8.7, 4.5 Hz, 2H), 6.58 (d, *J* = 8.3 Hz, 1H), 4.94 (d, *J* = 9.8 Hz, 1H), 4.83 (s, 1H), 4.67 (s, 1H), 4.54-4.42 (m, 2H), 3.91 (s, 3H), 3.90 (s, 3H), 3.88 (s, 8H), 3.43-3.29 (m, 2H), 3.26-3.18 (m, 8H), 1.58 (s, 4H), 1.51 (s, 9H), 1.42 (s, 9H). ¹³C-NMR (100 MHz, CDCl₃) δ (ppm): 168.92, 168.63, 155.54, 151.91, 147.55, 147.17, 141.35, 136.51, 133.86, 133.49, 131.06, 130.47, 130.41, 129.76, 129.71, 127.42, 127.23, 123.13, 122.78, 122.67, 120.23, 120.12, 118.97, 118.80, 118.73, 117.90, 117.53, 114.42, 114.28, 112.75, 112.54, 112.47, 111.49, 111.42, 110.27, 110.13, 80.80, 77.36, 67.10, 52.62, 52.13, 52.00, 49.68, 48.60, 48.48, 28.66, 28.47, 28.39. LC-MS (m/z): 632.38 ([M+Na]⁺). |
| (Ih) | ¹H-NMR (400 MHz, CDCl₃) δ (ppm): 8.42 (d, *J* = 8.1 Hz, 1.5H), 8.28 (s, 1H), 8.24 (s, 0.5H), 8.00 (dd, *J* = 8.0, 1.4 Hz, 0.5H), 7.96 (d, *J* = 7.7 Hz, 1H), 7.70 (d, *J* = 7.7 Hz, 1H), 7.58 (d, *J* = 7.9 Hz, 0.5H), 7.40 (dt, J *=* 17.1, 7.3 Hz, 2H), 7.32 (t, *J* = 7.7 Hz, 1.5H), 7.25-7.18 (m, 2H), 7.18-7.02 (m, 3.5H), 6.76 (dt, *J* = 17.6, 7.6 Hz, 2H), 6.51 (d, *J* = 8.3 Hz, 1H), 4.94 (d, *J* = 9.6 Hz, 1H), 4.88-4.75 (m, 1H), 4.69-4.60 (m, 1H), 4.46 (dd, *J* = 13.4, 5.4 Hz, 2H), 4.32 (dtd, *J* = 9.9, 7.0, 3.0 Hz, 3.5H), 3.34 (ddd, *J* = 28.3, 14.8, 5.5 Hz, 1.5H), 3.19 (dd, *J* = 14.5, 8.4 Hz, 2H), 1.51 (s, 9H), 1.41 (s, 4.5H), 1.40 (d, *J* = 2.7 Hz, 2H), 1.38 (d, *J* = 2.6 Hz, 2.5H), 1.36 (s, 1.5H). ¹³C-NMR (100 MHz, CDCl₃) δ (ppm): 168.53, 168.23, 155.50, 148.28, 147.87, 136.45, 134.63, 131.89, 127.16, 123.18, 122.57, 122.47, 120.02, 119.91, 118.85, 118.70, 118.01, 117.61, 117.50, 117.42, 112.67, 111.92, 111.49, 110.00, 80.69, 60.89, 60.73, 52.57, 48.17, 28.51, 28.41, 28.33, 14.36. LC-MS (m/z): 485.28 ([M+Na]⁺). |
| (Ii) | ¹H-NMR (400 MHz, CDCl₃) δ (ppm): 8.46 (d, *J* = 8.2 Hz, 1H), 8.39 (d, *J* = 7.4 Hz, 0.5H), 8.19 (s, 1H), 8.16 (s, 0.5H), 7.78 (dd, *J* = 12.1, 8.6 Hz, 1.5H), 7.69 (d, *J* = 7.9 Hz, 1H), 7.60 (d, *J* = 7.9 Hz, 0.5H), 7.40 (t, *J =* 7.2 Hz, 1.5H), 7.25-7.20 (m, 1.5H), 7.20-7.06 (m, 3.5H), 6.96 (s, 0.5H), 6.93-6.82 (m, 2H), 6.61 (s, 1H), 4.91 (d, *J* = 9.6 Hz, 1H), 4.87-4.78 (m, 0.5H), 4.70-4.58 (m, 1.5H), 4.47 (ddd, *J* = 13.5, 7.9, 5.5 Hz, 0.5H), 4.36 (dd, *J* = 7.7, 4.1 Hz, 1H), 3.87 (s, 1.5H), 3.84 (s, 3H), 3.33 (td, *J* = 15.8, 5.6 Hz, 1.5H), 3.18 (dd, *J* = 14.6, 8.9 Hz, 2H), 2.35-2.06 (m, 1.5H), 2.00 (s, 0.5H), 1.50 (s, 9H), 1.41 (s, 4.5H). ¹³C-NMR (100 MHz, CDCl₃) δ (ppm): 168.42, 168.14, 155.50, 149.06, 148.66, 136.51, 133.17, 130.10, 129.73, 127.06, 123.14, 122.81, 122.70, 120.88, 120.76, 120.25, 120.14, 118.87, 117.59, 114.79, 111.55, 111.27, 109.99, 109.82, 80.90, 77.36, 52.46, 52.20, 52.04, 48.26, 48.17, 28.79, 28.44, 28.35. LC-MS (m/z): 551.12 ([M+Na]⁺). |
| (Ij) | ¹H-NMR (400 MHz, CDCl₃) δ (ppm): 8.35-8.20 (m, 1.5H), 8.16-8.03 (m, 4H), 7.66-7.57 (m, 2H), 7.53-7.45 (m, 2H), 7.45-7.27 (m, 9H), 7.14 (dd, *J* = 16.8, 8.8 Hz, 2.5H), 7.10-6.98 (m, 3.5H), 6.83 (d, *J* = 8.7 Hz, 0.5H), 6.50 (d, *J* = 8.6 Hz, 1H), 4.87 (d, *J* = 9.6 Hz, 1H), 4.76 (d, *J* = 7.5 Hz, 1H), 4.64-4.53 (m, 1H), 4.46-4.31 (m, 2H), 3.82 (s, 1.5H), 3.79 (s, 3H), 3.28 (ddd, *J* = 26.9, 14.9, 5.3 Hz, 1.5H), 3.13 (dd, *J* = 14.6, 8.5 Hz, 2H), 1.43 (s, 7.5H), 1.34 (s, 5.5H), 1.31 (s, 2H), 1.28 (s, 4.5H), 1.27 (s, 9H), 1.25 (s, 1.5H). ¹³C-NMR (100 MHz, CDCl₃) δ (ppm): 168.97, 168.67, 155.55, 155.54, 149.96, 149.90, 147.31, 146.91, 137.21, 136.47, 135.70, 133.69, 133.56, 133.19, 130.49, 130.43, 130.23, 130.18, 127.19, 126.17, 125.85, 125.08, 123.16, 122.71, 122.60, 120.16, 120.05, 118.94, 118.77, 117.97, 117.58, 112.72, 112.54, 112.47, 111.49, 111.43, 110.14, 110.01, 80.79, 52.60, 52.11, 51.97, 48.50, 48.40, 34.61, 31.48, 31.34, 28.60, 28.46, 28.36. LC-MS (m/z): 603.32 ([M+Na]⁺). |
| (Ik) | ¹H-NMR (400 MHz, CDCl₃) δ (ppm): 8.27 (d, *J* = 5.7 Hz, 1.5H), 7.66 (d, *J* = 7.9 Hz, 1H), 7.60 (d, *J* = 8.0 Hz, 0.5H), 7.38 (d, *J* = 8.1 Hz, 1.5H), 7.27-7.19 (m, 2H), 7.14 (dtd, *J* = 13.9, 5.4, 1.9 Hz, 3.5H), 7.09-6.96 (m, 3.5H), 6.70 (d, *J* = 8.2 Hz, 0.5H), 6.47-6.33 (m, 2H), 6.27 (d, *J* = 7.9 Hz, 0.5H), 4.98 (d, *J* = 8.9 Hz, 1H), 4.86 (d, *J* = 7.7 Hz, 0.5H), 4.51 (tdd, *J* = 10.5, 8.1, 7.2, 3.9 Hz, 1.5H), 4.37 (ddd, *J* = 7.7, 6.0, 1.6 Hz, 0.5H), 4.23 (dd, J = 7.7, 3.5 Hz, 1H), 3.93 (s, 3H), 3.91 (s, 1.5H), 3.30 (ddd, J = 39.4, 15.0, 5.9 Hz, 2H), 3.15 (d, *J* = 9.1 Hz, 1H), 3.12 (d, *J* = 9.1 Hz, 0.5H), 1.50 (s, 9H), 1.42 (s, 4.5H). ¹³C-NMR (100 MHz, CDCl₃) δ (ppm): 167.67, 167.48, 156.18, 155.77, 146.01, 145.26, 136.54, 136.48, 132.70, 132.28, 127.30, 127.03, 124.20, 124.17, 123.76, 123.35, 123.14, 122.78, 122.69, 122.38, 122.01, 120.22, 120.16, 119.96, 119.37, 118.65, 118.57, 117.83, 117.13, 111.58, 111.15, 110.80, 109.89, 109.55, 81.13, 80.83, 77.36, 53.11, 52.56, 52.51, 50.15, 49.59, 28.72, 28.37. LC-MS (m/z): 551.22 ([M+Na]⁺). |
| (Il) | ¹H-NMR (400 MHz, CDCl₃) δ (ppm): 8.36 (t, *J* = 8.4 Hz, 1.5H), 8.24-8.15 (m, 3H), 7.75-7.65 (m, 1.5H), 7.62-7.54 (m, 2H), 7.46 (dd, *J* = 14.0, 8.1 Hz, 3.5H), 7.39 (t, *J* = 7.8 Hz, 2H), 7.30-7.07 (m, 10H), 6.92 (d, *J* = 7.3 Hz, 0.5H), 6.59 (d, *J* = 8.7 Hz, 1H), 4.95 (d, *J* = 9.6 Hz, 1H), 4.84 (s, 1H), 4.74-4.61 (m, 1H), 4.49 (ddd, *J* = 15.5, 7.8, 4.5 Hz, 2H), 3.91 (s, 1.5H), 3.88 (s, 3H), 3.36 (ddd, *J* = 27.0, 14.9, 5.3 Hz, 1.5H), 3.21 (dd, *J* = 14.6, 8.5 Hz, 2H), 2.69 (p, *J* = 7.4 Hz, 3.5H), 1.52 (s, 9H), 1.47 (s, 0.5H), 1.45 (s, 1H), 1.42 (s, 4.5H), 1.28 (td, *J* = 7.6, 5.2 Hz, 6H). ¹³C-NMR (100 MHz, CDCl₃) δ (ppm): 168.95, 168.66, 155.55, 147.29, 146.90, 143.12, 143.05, 137.46, 136.48, 133.21, 133.18, 130.54, 130.16, 130.10, 128.47, 128.43, 127.19, 126.44, 123.17, 122.70, 122.59, 120.14, 120.04, 118.92, 117.96, 117.56, 112.73, 112.56, 112.49, 111.50, 111.43, 110.13, 80.79, 77.36, 52.61, 52.10, 51.96, 48.51, 48.42, 28.62, 28.36, 15.79, 1.15. LC-MS (m/z): 575.43 ([M+Na]⁺). |
| (Im1) | ¹H-NMR (400 MHz, CDCl₃) δ (ppm): 8.36 (t, *J* = 8.5 Hz, 1.5H), 8.28-8.14 (m, 3H), 7.75-7.52 (m, 3.5H), 7.41 (dq, *J* = 15.7, 7.9 Hz, 5H), 7.22 (q, *J* = 7.9 Hz, 2H), 7.13 (p, *J* = 10.1, 8.9 Hz, 6.5H), 6.91 (d, *J* = 6.2 Hz, 0.5H), 6.58 (d, *J* = 8.5 Hz, 1H), 4.96 (d, *J* = 9.5 Hz, 1H), 4.90-4.79 (m, 1H), 4.67 (s, 1H), 4.54-4.43 (m, 1.5H), 3.91 (s, 1.5H), 3.88 (s, 3H), 3.36 (ddd, *J =* 26.1, 14.4, 4.8 Hz, 1.5H), 3.21 (dd, *J* = 14.5, 8.5 Hz, 1.5H), 2.32 (dd, *J* = 12.6, 6.2 Hz, 1H), 1.93 (td, *J* = 8.3, 5.2 Hz, 1.5H), 1.52 (s, 9H), 1.47 (s, 1H), 1.46 (s, 1H), 1.43 (s, 4.5H), 0.99 (ddt, *J* = 8.5, 6.1, 3.2 Hz, 3H), 0.73 (t, *J* = 5.4 Hz, 3H). ¹³C-NMR (100 MHz, CDCl₃) δ (ppm): 168.93, 168.64, 168.63, 155.54, 155.52, 147.25, 146.85, 142.89, 142.82, 137.18, 136.46, 133.46, 133.09, 130.43, 130.03, 127.74, 127.17, 126.35, 126.20, 126.16, 123.84, 123.18, 122.67, 122.57, 120.12, 120.01, 118.90, 118.73, 117.96, 117.94, 117.58, 117.55, 112.72, 112.55, 112.49, 111.51, 111.44, 110.08, 109.95, 80.77, 52.59, 52.10, 51.95, 48.50, 48.41, 28.59, 28.44, 28.35, 20.05, 19.52, 15.20, 9.43. LC-MS (m/z): 587.38 ([M+Na]⁺). |
| (Im2) | ¹H-NMR (400 MHz, CDCl₃) δ (ppm): 8.35 (t, *J* = 8.2 Hz, 1.5H), 8.18-8.12 (m, 2H), 7.71 (d, *J* = 7.9 Hz, 1H), 7.61-7.52 (m, 1.5H), 7.47-7.36 (m, 4H), 7.22 (dt, *J* = 8.6, 1.5 Hz, 1.2H), 7.20-7.07 (m, 5H), 6.91 (d, *J* = 8.6 Hz, 0.2H), 6.58 (d, *J* = 8.7 Hz, 1H), 4.92 (d, *J* = 9.8 Hz, 1H), 4.82 (s, 0.4H), 4.66 (s, 1H), 4.46 (dd, *J* = 8.0, 4.4 Hz, 1.5H), 3.91 (s, 0.66H), 3.88 (s, 3H), 3.33 (dd, *J* = 14.8, 6.1 Hz, 1.6H), 3.21 (dd, *J* = 14.7, 8.5 Hz, 1.6H), 2.31 (dd, *J* = 12.8, 6.4 Hz, 1H), 1.92 (tt, *J* = 8.4, 5.2 Hz, 1.2H), 1.51 (s, 9H), 1.41 (s, 1.8H), 1.26 (s, 2.4H), 1.02-0.93 (m, 2H), 0.92-0.80 (m, 1.6H), 0.75-0.67 (m, 2.6H). LC-MS (m/z): 587.38 ([M+Na]⁺). |
| (Im3) | ¹H-NMR (400 MHz, CDCl₃) δ (ppm): 8.35 (t, *J=* 8.2 Hz, 1.33H), 8.19 (d, J = 2.3 Hz, 0.33H), 8.18-8.12 (m, 2H), 8.11 (s, 0.33H), 7.71 (d, *J* = 7.9 Hz, 1H), 7.68 (s, 0.66H), 7.61-7.52 (m, 1.66H), 7.47-7.36 (m, 3.66H), 7.22 (dt, *J* = 8.6, 1.5 Hz, 1.33H), 7.20-7.07 (m, 5H), 6.91 (d, *J* = 8.6 Hz, 0.33H), 6.58 (d, *J* = 8.7 Hz, 1H), 4.92 (d, *J* = 9.8 Hz, 1H), 4.90-4.79 (m, 0.33H), 4.66 (s, 1H), 4.46 (dd, J = 8.0, 4.4 Hz, 1.33H), 3.90 (s, 1H), 3.88 (s, 3H), 3.33 (dd, *J* = 14.8, 6.1 Hz, 1.33H), 3.21 (dd, *J* = 14.7, 8.5 Hz, 1.66H), 2.31 (dd, *J* = 12.8, 6.4 Hz, 1H), 1.92 (tt, *J* = 8.4, 5.2 Hz, 1.33H), 1.51 (s, 9H), 1.41 (s, 3 H), 1.26 (s, 2H), 1.02-0.93 (m, 2H), 0.92-0.80 (m, 1.33H), 0.75-0.67 (m, 2.33H). LC-MS (m/z): 587.48 ([M+Na]⁺). |
| (Im4) | ¹H-NMR (400 MHz, CDCl₃) δ (ppm): 8.35 (t, *J* = 8.1 Hz, 2H), 8.19 (d, *J* = 2.3 Hz, 1H), 8.17-8.12 (m, 1H), 8.12-8.07 (m, 1H), 7.71 (d, *J* = 8.1 Hz, 0.5H), 7.67 (dd, *J* = 8.7, 2.4 Hz, 1.5H), 7.57 (dd, *J* = 13.5, 8.3 Hz, 2H), 7.47-7.35 (m, 5.5H), 7.25-7.16 (m, 3H), 7.16-7.07 (m, 6H), 6.91 (d, *J =* 8.7 Hz, 1H), 6.58 (d, *J* = 8.7 Hz, 0.5H), 4.96 (d, *J* = 9.6 Hz, 0.5H), 4.82 (s, 2.5H), 4.66 (s, 1H), 4.49 (tt, *J* = 7.8, 5.1 Hz, 2H), 3.90 (s, 3H), 3.88 (s, 1.5H), 3.44-3.27 (m, 2H), 3.27-3.13 (m, 2H), 2.31 (dd, *J* = 12.8, 6.4 Hz, 1H), 1.92 (tq, *J* = 8.4, 5.4 Hz, 2H), 1.51 (s, 4.5H), 1.41 (s, 9H), 1.26 (s, 3H), 1.03-0.94 (m, 3H), 0.92-0.79 (m, 2H), 0.73 (dt, *J* = 6.4, 4.5 Hz, 3H). LC-MS (m/z): 587.38 ([M+Na]⁺). |
| (In) | ¹H-NMR (400 MHz, CDCl₃) δ (ppm): 8.31 (t, *J* = 7.6 Hz, 2H), 8.24 (s, 1.5H), 8.22-8.10 (m, 2H), 8.06 (s, 0.66H), 7.62 (ddd, *J* = 44.0, 23.7, 8.2 Hz, 5H), 7.42 (ddd, *J* = 20.4, 12.4, 5.0 Hz, 5H), 7.27-7.06 (m, 6H), 6.97 (t, *J* = 8.5 Hz, 3H), 6.89 (d, *J* = 8.3 Hz, 0.66H), 6.72 (d, *J* = 8.5 Hz, 0.66H), 6.57 (d, *J* = 8.7 Hz, 1H), 4.94 (d, *J* = 9.4 Hz, 1H), 4.82 (s, 0.66H), 4.66 (s, 1H), 4.46 (d, *J* = 7.3 Hz, 1.66H), 3.90 (d, *J* = 2.9 Hz, 2H), 3.87 (s, 3H), 3.48 (s, 0.66H), 3.40-3.15 (m, 10H), 2.62 (d, *J* = 5.0 Hz, 10H), 2.38 (s, 7.5H), 1.50 (s, 9H), 1.44 (s, 2H), 1.41 (s, 6H), 1.25 (s, 2.66H), 0.91-0.76 (m, 1.66H). ¹³C-NMR (100 MHz, CDCl₃) δ (ppm): 168.99, 168.70, 155.54, 150.22, 149.29, 146.95, 146.56, 136.51, 133.19, 132.81, 132.59, 131.48, 130.41, 129.66, 129.58, 128.89, 127.23, 127.16, 127.02, 123.15, 122.72, 122.62, 120.17, 120.07, 118.96, 118.80, 118.00, 117.62, 117.34, 116.42, 112.79, 112.62, 112.53, 111.47, 111.42, 110.22, 110.10, 80.76, 77.36, 55.10, 52.62, 52.09, 51.95, 51.74, 49.03, 48.56, 48.46, 46.15, 41.12, 29.84, 28.46, 28.38. LC-MS (m/z): 623.54 ([M+H]⁺). |
| (Io) | ¹H-NMR (400 MHz, CDCl₃) δ (ppm): 8.34 (d, *J* = 8.6 Hz, 1.33H), 8.23 (s, 1H), 8.18 (d, *J* = 2.4 Hz, 0.33H), 8.16 (d, *J* = 2.3 Hz, 1H), 7.79 (s, 1H), 7.66 (dd, *J* = 10.8, 2.1 Hz, 0.33H), 7.59 (dd, *J* = 8.6, 2.4 Hz, 1.33H), 7.47 (t, *J* = 8.9 Hz, 2.66H), 7.32 (d, *J* = 1.8 Hz, 0.33H), 7.30 (d, *J* = 1.8 Hz, 1H), 7.26 (d, *J* = 8.7 Hz, 3.66H), 7.16 (d, *J* = 2.6 Hz, 1.33H), 6.97 (d, *J* = 8.3 Hz, 2.66H), 6.63 (d, *J* = 8.9 Hz, 1.33H), 4.90 (d, *J* = 9.7 Hz, 1H), 4.81 (s, 0.33H), 4.59 (s, 1H), 4.50-4.37 (m, 2H), 3.92 (s, 1H), 3.88 (d, *J* = 7.1 Hz, 9.5H), 3.39-3.23 (m, 1.66H), 3.19 (q, *J* = 4.8 Hz, 7H), 3.14 (d, *J* = 7.6 Hz, 0.66H), 1.49 (s, 9H), 1.43 (d, *J* = 2.3 Hz, 3H). ¹³C-NMR (100 MHz, CDCl₃) δ (ppm): 169.08, 168.77, 155.39, 150.38, 146.88, 146.60, 135.11, 132.97, 131.79, 130.55, 129.77, 129.11, 127.27, 125.69, 124.40, 121.54, 117.80, 117.50, 116.10, 113.52, 112.91, 112.54, 110.10, 110.03, 80.86, 77.36, 67.01, 52.55, 52.03, 49.47, 48.55, 41.16, 28.44, 28.18. LC-MS (m/z): 690.25 ([M+H]⁺). |
| (lp) | ¹H-NMR (400 MHz, CDCl₃) δ (ppm): 8.45 (d, *J* = 8.3 Hz, 1H), 8.42 (d, *J* = 7.8 Hz, 0.77H), 8.17 (d, *J* = 2.2 Hz, 2H), 8.13 (s, 2H), 7.71 (d, *J* = 7.6 Hz, 0.77H), 7.64 (dt, *J* = 8.7, 2.0 Hz, 1H), 7.59 (d, *J* = 7.9 Hz, 1H), 7.56-7.51 (m, 1H), 7.41-7.36 (m, 2.54H), 7.26-7.19 (m, 2.54H), 7.18-7.08 (m, 5H), 7.06-6.98 (m, 2.77H), 6.90 (ddd, *J* = 12.3, 6.5, 3.1 Hz, 3H), 6.77 (m, 1.77H), 6.58 (d, *J* = 9.2 Hz, 1H), 4.93 (d, *J* = 9.5 Hz, 1H), 4.82 (s, 1.77H), 4.67 (s, 1H), 4.56-4.44 (m, 4H), 3.89 (s, 2.3H), 3.87 (s, 3H), 3.42-3.26 (m, 2.77H), 3.23 (d, *J* = 8.4 Hz, 2H), 3.19 (d, *J* = 8.7 Hz, 1H), 1.51 (s, 7H), 1.45 (s, 2H), 1.42 (s, 9H), 1.36-1.32 (m, 11.6H). ¹³C-NMR (100 MHz, CDCl₃) δ (ppm): 168.83, 168.54, 155.52, 155.47, 154.19, 153.10, 147.73, 147.35, 136.51, 135.56, 135.25, 135.21, 132.33, 132.30, 128.69, 128.55, 127.41, 127.21, 125.19, 125.10, 123.14, 122.78, 122.67, 120.22, 120.12, 118.95, 118.79, 117.86, 117.78, 117.75, 116.81, 116.77, 116.57, 116.53, 115.68, 115.62, 115.60, 115.54, 112.50, 112.15, 112.04, 111.50, 111.43, 110.22, 110.07, 80.80, 77.36, 71.00, 52.66, 52.13, 51.99, 48.46, 48.34, 28.47, 28.37, 22.19. LC-MS (m/z): 623.22 ([M+Na]⁺). |
| (Iq) | ¹H-NMR (400 MHz, CDCl₃) 8.44 (s, 1H), 8.37 (s, 1H), 8.36 (d, *J* = 3.3 Hz, 1H), 8.33-8.27 (m, 3.5H), 8.11 (d, *J* = 2.3 Hz, 1H), 8.08 (d, *J* = 2.4 Hz, 1H), 7.70 (d, *J* = 7.6 Hz, 1H), 7.66-7.61 (m, 2H), 7.60-7.55 (m, 2.5H) 7.46 (dd, J = 8.7, 2.4 Hz, 1H) 7.38 (t, *J* = 7.8 Hz, 2.5H), 7.22 (qd, *J* = 7.6,1.5 Hz, 2.5H), 7.17-7.08 (m, 5H), 6.89 (d, *J* = 7.8 Hz, 1H), 6.56 (d, *J* = 8.8 Hz, 1H) 6.43 (t, *J* = 8.4 Hz, 2.3H), 4.95 (d, *J* = 9.4 Hz, 1H) 4.85 (s, 1H) 4.80 (s, 1H) 4.70-4.61 (m, 1.5H) 4.52-4.41 (m, 2.5H) 3.90 (s, 3H), 3.87 (s, 3.4H) 3.49 (ddd, 8.6H), 3.20 (q, *J* = 8.6 Hz, 2.5H), 2.03 (h, *J* = 3.7 Hz, 9H), 1.51 (s, 9H), 1.41 (s, 8.5H) ¹³C-NMR (100 MHz, CDCl₃) δ (ppm): 168.87, 168.57, 156.10, 155.53, 146.92, 146.53, 145.34, 136.54 (d, *J* = 3.1 Hz), 135.56, 132.70, 132.32, 129.19, 129.14, 128.25, 128.14, 127.41, 127.21, 123.38, 123.20, 122.68, 122.57, 120.12, 120.02, 118.92, 118.75, 117.98, 112.95, 112.80, 112.73, 111.48 (d, *J* = 7.3 Hz), 110.11, 109.99, 106.79, 80.74, 52.61, 52.10, 51.96, 48.56, 48.47, 47.05, 28.45, 28.37, 25.67. LC-MS (m/z): 595.33 ([M+H]⁺). |
| (Ir) | ¹H-NMR (400 MHz, CDCl₃) δ (ppm): 8.68 (d, *J* = 3.3 Hz, 0.5H), 8.64 (d, *J* = 2.4 Hz, 1H), 8.43 (d, *J* = 8.4 Hz, 1H), 8.40 (d, *J* = 8.1 Hz, 0.5H), 8.28 (s, 1H), 8.24 (s, 0.5H), 8.18 (d, *J* = 2.3 Hz, 0.5H), 8.15 (d, *J* = 2.2 Hz, 1H), 7.71 (ddd, *J* = 13.2, 8.0, 2.4 Hz, 2.5H), 7.65 (dd, *J* = 8.6, 2.4 Hz, 0.5H), 7.58 (d, *J* = 7.9 Hz, 0.5H), 7.53 (d, *J* = 8.9 Hz, 1H), 7.42-7.36 (m, 1.5H), 7.24-7.22 (m, 0.5H), 7.21 (s, 1H), 7.20-7.16 (m, 2.5H), 7.15 (s, 1H), 7.14-7.08 (m, 1.5H), 6.96 (d, *J* = 7.2 Hz, 0.5H), 6.59 (d, *J* = 8.8 Hz, 1H), 4.95 (d, *J* = 9.8 Hz, 1H), 4.84 (s, 1H), 4.68 (s, 1H), 4.53-4.43 (m, 1.5H), 3.91 (s, 2H), 3.88 (s, 3H), 3.43-3.29 (m, 1.5H), 3.22 (d, *J* = 8.7 Hz, 1H), 3.19 (d, *J* = 8.7 Hz, 0.5H), 2.60 (s, 2H), 2.58 (s, 3H), 1.51 (s, 9H), 1.42 (s, 6H). ¹³C-NMR (100 MHz, CDCl₃) δ (ppm): 168.69, 168.40, 156.83, 156.77, 155.54, 147.86, 147.48, 146.94, 136.54, 134.21, 133.42, 133.04, 132.80, 130.27, 130.22, 127.19, 127.12, 123.34, 123.16, 122.79, 122.67, 120.22, 120.10, 118.95, 118.75, 117.79, 117.40, 112.98, 112.85, 112.78, 111.53, 111.45, 110.18, 110.02, 80.84, 77.36, 52.57, 52.21, 52.07, 48.53, 48.43, 28.73, 28.47, 28.38, 24.17, 24.15. LC-MS (m/z): 540.25 ([M+H]⁺). |
| (Is) | ¹H-NMR (400 MHz, CDCl₃) δ (ppm): 8.41 (d, *J* = 1.8 Hz, 0.5H), 8.37 (d, *J* = 2.3 Hz, 1.5H), 8.35 (s, 1H), 8.24 (d, *J* = 2.6 Hz, 1H), 8.19 (s, 0.5H), 8.13 (d, *J* = 2.3 Hz, 0.5H), 8.09 (d, *J* = 2.4 Hz, 1H), 7.69 (ddd, *J* = 12.1, 8.8, 2.6 Hz, 3H), 7.62-7.56 (m, 1.5H), 7.48 (d, *J* = 8.7 Hz, 1H), 7.39 (t, *J* = 8.1 Hz, 1.5H), 7.25-7.20 (m, 2H), 7.19-7.08 (m, 4H), 6.92 (d, *J* = 7.9 Hz, 0.5H), 6.70 (t, *J* = 8.4 Hz, 2H), 6.57 (d, *J* = 8.4 Hz, 1H), 4.94 (d, *J =* 10.6 Hz, 1H), 4.83 (s, 1H), 4.67 (s, 1H), 4.53-4.42 (m, 1.5H), 3.90 (s, 1.5H), 3.87 (s, 3H), 3.87-3.81 (m, 6H), 3.55 (q, *J* = 5.3 Hz, 6H), 3.42 (t, *J* = 5.0 Hz, 1H), 3.39-3.28 (m, 1.5H), 3.22 (d, *J* = 8.7 Hz, 1H), 3.18 (d, *J* = 8.6 Hz, 0.5H), 1.51 (s, 9H), 1.41 (s, 4.5H). ¹³C-NMR (100 MHz, CDCl₃) δ (ppm): 168.77, 168.48, 155.54, 147.28, 146.89, 136.49, 136.13, 132.87, 132.48, 129.51, 129.46, 127.38, 127.17, 126.05, 123.16, 122.76, 122.65, 120.19, 120.08, 118.95, 118.76, 117.89, 117.50, 112.94, 112.83, 112.76, 112.60, 111.51, 111.44, 110.16, 110.01, 107.20, 80.82, 77.36, 66.83, 66.62, 52.53, 52.17, 52.03, 48.52, 48.42, 47.04, 45.95, 28.67, 28.45, 28.37. LC-MS (m/z): 611.37 ([M+H]⁺). |
| (It) | ¹H-NMR (400 MHz, CDCl₃) δ (ppm): 8.39 (d, *J* = 8.4 Hz, 1H), 8.36 (d, *J* = 8.2 Hz, 0.77H), 8.33 (dt, *J* = 3.1, 1.5 Hz, 1H), 8.30 (d, *J* = 2.6 Hz, 1H), 8.20 (s, 1H), 8.16 (s, 0.77H), 8.13 (d, *J* = 2.3 Hz, 0.77H), 8.10 (d, *J* = 2.4 Hz, 1H), 7.73 (ddd, *J* = 12.8, 8.6, 2.6 Hz, 2.54H), 7.63-7.56 (m, 1.77H), 7.52-7.43 (m, 1.54H), 7.39 (t, *J* = 8.2 Hz, 2H), 7.26-7.20 (m, 2H), 7.20-7.08 (m, 5H), 6.94 (d, *J* = 7.9 Hz, 0.77H), 6.83-6.76 (m, 2H), 6.58 (d, *J* = 8.4 Hz, 1H), 4.94 (d, *J* = 9.8 Hz, 1H), 4.83 (s, 1H), 4.67 (s, 1H), 4.47 (ddd, *J* = 14.1, 8.5, 3.3 Hz, 2H), 3.98 (s, 2.3H), 3.97 (d, *J* = 2.2 Hz, 3H), 3.90 (d, *J* = 2.8 Hz, 2.3H), 3.88 (s, 3H), 3.43-3.25 (m, 2.54H), 3.22 (d, *J* = 8.6 Hz, 1H), 3.19 (d, *J* = 8.4 Hz, 0.77H), 1.61 (s, 3H), 1.51 (s, 9H), 1.44 (s, 2H), 1.42 (s, 7H). LC-MS (m/z): 556.19 ([M+H]⁺). |
| (Iu) | ¹H-NMR (400 MHz, CDCl₃) δ (ppm): 8.36 (d, *J* = 8.2 Hz, 1.3H), 8.22 (d, *J* = 14.3 Hz, 1.3H), 8.00- 7.90 (m, 1.3H), 7.70 (d, *J* = 7.7 Hz, 1H), 7.58 (d, *J* = 7.9 Hz, 0.3H), 7.37 (tq, *J* = 15.3, 7.5 Hz, 3H), 7.28-7.17 (m, 2H), 7.17-7.08 (m, 2H), 7.07 (s, 0.3H), 6.78 (dq, *J* = 24.7, 7.5 Hz, 2H), 6.52 (d, *J* = 8.3 Hz, 1H), 4.94 (d, *J* = 9.2 Hz, 1H), 4.80 (d, *J* = 28.0 Hz, 1H), 4.64 (s, 1H), 4.46 (ddd, *J* = 19.4, 7.8, 4.8 Hz, 1H), 3.89 (s, 1H), 3.86 (s, 3H), 3.40-3.25 (m, 2H), 3.20 (dd, *J* = 14.6, 8.3 Hz, 1.3H), 1.51 (s, 9H), 1.41 (s, 3H), 1.29-1.18 (m, 2H). ¹³C-NMR (100 MHz, CDCl₃) δ (ppm): 168.95, 168.65, 155.53, 148.28, 147.87, 136.48, 135.14, 134.77, 131.95, 127.37, 127.19, 123.15, 122.66, 122.56, 120.10, 120.00, 118.90, 118.74, 117.58, 117.50, 112.45, 111.97, 111.48, 110.12, 109.98, 80.75, 77.36, 52.59, 52.01, 51.87, 48.39, 48.26, 28.58, 28.43, 28.35, 15.40. LC-MS (m/z): 471.23 ([M+Na]⁺). |
| (Iv) | ¹H-NMR (400 MHz, CDCl₃) δ (ppm): 8.49 (dd, *J* = 12.9, 8.4 Hz, 1.5H), 8.25 (s, 1H), 8.21 (s, 0.5H), 7.91 (dd, *J* = 11.6, 4.5 Hz, 2H), 7.85-7.75 (m, 0H), 7.71 (d, *J* = 7.8 Hz, 1H), 7.63 (d, *J* = 7.9 Hz, 1H), 7.44-7.35 (m, 2H), 7.35-7.27 (m, 2H), 7.25-7.17 (m, 2H), 7.18-6.96 (m, 5H), 6.91-6.80 (m, 1H), 6.74 (dt, *J* = 19.2, 7.5 Hz, 2H), 6.49 (d, *J* = 8.3 Hz, 1H), 6.37 (dt, *J* = 24.9, 7.7 Hz, 0.5H), 4.95 (d, *J* = 9.6 Hz, 1H), 4.88-4.75 (m, 1H), 4.71-4.58 (m, 1H), 4.42 (dt, *J* = 8.2, 4.8 Hz, 2H), 3.55 (s, 0.5H), 3.49-3.26 (m, 2H), 3.19 (dd, *J* = 14.5, 8.5 Hz, 2H), 1.66 (s, 1.5H), 1.60 (d, *J* = 1.9 Hz, 4.5H), 1.57 (s, 9H), 1.51 (s, 9H), 1.41 (s, 4.5H), 1.27 (s, 1.5H). ¹³C-NMR (100 MHz, CDCl₃) δ (ppm): 167.73, 155.44, 148.37, 147.93, 136.48, 134.21, 132.13, 127.25, 123.13, 122.63, 122.52, 120.08, 119.96, 118.93, 117.72, 117.37, 117.28, 114.10, 111.87, 111.46, 110.22, 81.32, 80.61, 52.66, 48.17, 29.83, 28.63, 28.58, 28.55, 28.51, 28.40, 28.35. LC-MS (m/z): 513.37 ([M+Na]⁺). |
| (Iw) | ¹H-NMR (400 MHz, CDCl₃) δ (ppm): 8.27 (t, *J* = 8.3 Hz, 1.5H), 8.16-8.07 (m, 3H), 7.66-7.56 (m, 1.5H), 7.49 (t, *J* = 9.2 Hz, 1.5H), 7.41-7.26 (m, 5H), 7.19-7.09 (m, 5H), 7.04 (dt, *J*= 16.2, 7.3 Hz, 3.5H), 6.83 (d, *J* = 7.9 Hz, 0.5H), 6.50 (d, *J* = 8.6 Hz, 1H), 4.87 (d, *J* = 9.5 Hz, 1H), 4.76 (s, 1H), 4.59 (s, 1H), 4.41 (ddt, *J* = 10.9, 7.8, 4.3 Hz, 2H), 3.82 (s, 1.5H), 3.79 (s, 3H), 3.27 (ddd, *J* = 27.1, 14.9, 5.4 Hz, 1.5H), 3.12 (dd, *J* = 14.6, 8.5 Hz, 1.5H), 2.56 (q, *J* = 7.6, 6.8 Hz, 3H), 1.61-1.48 (m, 4H), 1.43 (s, 9H), 1.34 (s, 4.5H), 1.33-1.23 (m, 4H), 0.86 (td, *J* = 7.3, 3.6 Hz, 5H). ¹³C-NMR (100 MHz, CDCl₃) δ (ppm): 168.96, 168.67, 168.66, 155.55, 147.29, 146.89, 141.77, 141.71, 137.40, 136.48, 133.55, 133.17, 130.56, 130.15, 130.10, 129.03, 128.98, 127.19, 126.35, 123.17, 123.16, 122.70, 122.59, 120.15, 120.04, 118.93, 118.76, 117.99, 117.97, 117.59, 117.58, 112.73, 112.56, 112.55, 112.49, 112.48, 111.51, 111.49, 111.44, 110.13, 110.12, 109.99, 80.78, 77.36, 52.60, 52.10, 51.96, 48.51, 48.41, 35.37, 33.81, 28.61, 28.46, 28.37, 22.50, 14.12. LC-MS (m/z): 603.42 ([M+Na]⁺). |
| (Ix) | ¹H-NMR (400 MHz, CDCl₃) δ (ppm): 8.31-8.23 (m, 1.5H), 8.23-8.10 (m, 3.5H), 7.71 (d, *J* = 8.0 Hz, 1H), 7.67-7.50 (m, 3H), 7.41 (dd, *J* = 13.3, 8.5 Hz, 5.5H), 7.22 (q, *J* = 7.7 Hz, 2.5H), 7.18-7.07 (m, 4H), 7.03 (s, 0.5H), 6.88 (d, *J* = 6.2 Hz, 1H), 6.74 (t, *J* = 8.3 Hz, 4H), 6.57 (d, *J* = 8.7 Hz, 1H), 4.93 (d, *J* = 8.3 Hz, 1H), 4.82 (s, 1H), 4.65 (s, 1.5H), 4.55-4.41 (m, 2H), 3.90 (s, 1.5H), 3.87 (s, 3H), 3.38 (q, *J* = 7.0, 6.1 Hz, 7H), 3.34-3.25 (m, 2H), 3.21 (dd, *J* = 14.6, 8.3 Hz, 2H), 1.51 (s, 9H), 1.45 (s, 3H), 1.42 (s, 4.5H), 1.23-1.15 (m, 9H). ¹³C-NMR (100 MHz, CDCl₃) δ (ppm): 155.54, 136.53, 127.17, 123.71, 123.22, 122.70, 122.59, 120.14, 120.03, 118.91, 118.72, 117.42, 112.83, 112.65, 111.56, 111.49, 110.07, 80.83, 77.36, 53.78, 52.58, 52.17, 52.04, 48.51, 48.42, 28.45, 28.37, 28.36, 28.31, 10.41. LC-MS (m/z): 596.45 ([M+H]⁺). |
| (Iy) | ¹H-NMR (400 MHz, CDCl₃) δ (ppm): 8.33 (t, *J* = 8.1 Hz, 1.66H), 8.24-8.11 (m, 3H), 7.62 (ddd, *J* = 42.9, 27.5, 7.9 Hz, 4H), 7.49-7.35 (m, 5.66H), 7.27-7.07 (m, 6.66H), 6.95 (t, *J* = 8.7 Hz, 4H), 6.57 (d, *J* = 8.6 Hz, 1H), 4.94 (d, *J* = 9.6 Hz, 1H), 4.83 (s, 1H), 4.67 (d, *J* = 3.5 Hz, 1H), 4.55-4.41 (m, 1.66H), 3.96 (q, *J* = 6.4 Hz, 3.33H), 3.91 (s, 2H), 3.88 (s, 3H), 3.36 (ddd, *J* = 25.5, 14.9, 5.2 Hz, 1.66H), 3.21 (dd, *J* = 14.4, 8.6 Hz, 2H), 1.83 (tt, *J* = 11.3, 5.6 Hz, 4H), 1.63 (s, 2H), 1.51 (s, 9H), 1.46 (t, *J* = 4.6 Hz, 3H), 1.42 (s, 6H), 1.06 (td, *J* = 7.4, 3.6 Hz, 5H). ¹³C-NMR (100 MHz, CDCl₃) δ (ppm): 168.97, 168.67, 158.50, 155.54, 147.03, 146.64, 136.50, 133.35, 132.96, 132.52, 130.46, 130.40, 129.83, 127.52, 127.37, 127.22, 123.15, 122.73, 122.62, 120.17, 120.07, 118.96, 118.78, 117.98, 117.60, 114.98, 112.78, 112.53, 111.49, 111.48, 110.21, 110.08, 80.77, 77.36, 69.74, 52.60, 52.10, 51.96, 48.56, 48.45, 41.10, 28.61, 28.46, 28.36, 22.74, 10.68. LC-MS (m/z): 605.41 ([M+Na]⁺). |
| (Iz) | ¹H-NMR (400 MHz, CDCl₃) δ (ppm): 8.37 (s, 1.5H), 8.20 (d, *J* = 2.3 Hz, 0.5H), 8.19-8.10 (m, 2.5H), 7.75-7.67 (m, 1.5H), 7.59 (td, *J* = 5.4, 2.6 Hz, 2H), 7.54 (s, 1H), 7.50 (s, 1.5H), 7.41 (s, 0.5H), 7.39 (s, 1H), 7.38-7.31 (m, 6H), 7.23 (d, *J* = 7.0 Hz, 1.5H), 7.20-7.15 (m, 2H), 7.14 (d, *J* = 2.3 Hz, 0.5H), 7.12-7.09 (m, 1H), 6.94 (d, *J* = 7.3 Hz, 0.5H), 6.60 (d*, J* = 8.4 Hz, 1H), 4.93 (d, *J* = 9.7 Hz, 1H), 4.83 (s, 1H), 4.68 (s, 1H), 4.54-4.44 (m, 2H), 3.92 (s, 1.5H), 3.89 (s, 3H), 3.37 (ddd, *J* = 26.3, 14.7, 5.3 Hz, 1.5H), 3.22 (dd, *J* = 14.7, 8.5 Hz, 2H), 1.51 (s, 9H), 1.42 (s, 4.5H), 1.38 (s, 4.5H), 1.37 (s, 9H). ¹³C-NMR (100 MHz, CDCl₃) δ (ppm): 168.98, 168.68, 155.54, 151.78, 147.43, 147.04, 139.95, 136.50, 133.95, 133.56, 131.27, 130.48, 130.43, 128.67, 128.63, 127.23, 124.08, 124.03, 123.91, 123.70, 123.14, 122.77, 122.66, 120.22, 120.11, 118.98, 118.80, 117.94, 117.57, 112.77, 112.55, 112.49, 111.49, 111.42, 110.26, 110.12, 80.80, 77.36, 52.61, 52.14, 52.00, 48.57, 48.46, 34.94, 31.55, 28.63, 28.47, 28.37. LC-MS (m/z): 603.30 ([M+Na]⁺). |
| (Iaa) | ¹H-NMR (400 MHz, CDCl₃) δ (ppm): 8.37 (t, *J* = 7.9 Hz, 1.66H), 8.22 (d, *J* = 2.3 Hz, 0.66H), 8.18 (d, *J* = 2.3 Hz, 1H), 8.16 (s, 1H), 8.13 (s, 0.66H), 7.74-7.67 (m, 1.66H), 7.61-7.55 (m, 2H), 7.41 (s, 0.66H), 7.39 (s, 1H), 7.39-7.35 (m, 2.66H), 7.35-7.31 (m, 4H), 7.24 (s, 0.66H), 7.22 (s, 0.66H), 7.17 (d, *J* = 6.6 Hz, 3H), 7.14 (d, *J* = 4.0 Hz, 1.32H), 7.11 (d, *J* = 7.0 Hz, 1.32H), 6.93 (d, *J* = 7.7 Hz, 0.66H), 6.59 (d, *J* = 8.6 Hz, 1H), 4.93 (d, *J* = 9.2 Hz, 1H), 4.83 (s, 1H), 4.67 (s, 1H), 4.54-4.44 (m, 2H), 3.92 (s, 2H), 3.89 (s, 3H), 3.36 (ddd, *J* = 26.5, 14.7, 5.4 Hz, 2H), 3.21 (dd, *J* = 14.7, 8.5 Hz, 2H), 2.71 (p, *J* = 7.5 Hz, 5H), 1.51 (s, 9H), 1.42 (s, 6H), 1.29 (td, *J* = 7.6, 6.3 Hz, 5H). ¹³C-NMR (100 MHz, CDCl₃) δ (ppm): 168.96, 168.67, 155.53, 147.46, 147.07, 144.98, 144.93, 140.15, 136.51, 133.78, 133.40, 130.93, 130.83, 130.38, 130.32, 128.95, 128.91, 127.24, 126.56, 126.51, 126.20, 123.95, 123.14, 122.77, 122.66, 120.21, 120.11, 118.97, 118.79, 117.93, 117.56, 112.78, 112.57, 112.49, 111.49, 111.43, 110.26, 110.12, 80.79, 77.36, 52.65, 52.12, 51.98, 48.58, 48.47, 29.14, 29.12, 28.47, 28.38, 15.84. LC-MS (m/z): 575.31 ([M+Na]⁺). |
| (Iab) | ¹H-NMR (400 MHz, CDCl₃) δ (ppm): 8.36 (t, *J* = 7.5 Hz, 1.66H), 8.22 (d, *J* = 2.3 Hz, 0.66H), 8.18 (s, 2H), 8.14 (s, 0.66H), 7.70 (dd, *J* = 8.7, 2.3 Hz, 1.66H), 7.59 (d, *J* = 7.9 Hz, 1.66H), 7.39 (t, *J* = 7.8 Hz, 2H), 7.32-7.26 (m, 2H), 7.26-7.19 (m, 3H), 7.17 (s, 2H), 7.15 (s, 0.66H), 7.12 (d, *J* = 7.9 Hz, 1H), 7.09 (d, *J* = 1.1 Hz, 0.66H), 6.88 (d, *J* = 18.3 Hz, 2.66H), 6.73 (d, *J* = 8.4 Hz, 1H), 6.58 (d, *J* = 8.9 Hz, 2H), 4.93 (d, *J* = 9.7 Hz, 1H), 4.82 (s, 1H), 4.67 (s, 1H), 4.54-4.43 (m, 2H), 3.91 (s, 2H), 3.88 (s, 3H), 3.34 (d, *J* = 19.3 Hz, 6.66H), 3.21 (dd, *J* = 14.7, 8.4 Hz, 1.66H), 2.05 (s, 6.66H), 1.51 (s, 9H), 1.46 (s, 0.66H), 1.45 (s, 1H), 1.42 (s, 6H). ¹³C-NMR (100 MHz, CDCl₃) δ (ppm): 169.01, 168.71, 155.52, 147.46, 147.08, 141.31, 136.51, 133.95, 133.58, 130.50, 130.44, 129.72, 127.42, 127.25, 123.14, 122.75, 122.64, 120.21, 120.10, 118.97, 118.80, 117.96, 117.60, 112.69, 112.44, 112.37, 111.49, 111.42, 110.25, 110.13, 80.76, 77.36, 52.66, 52.10, 51.96, 48.57, 48.46, 28.60, 28.47, 28.39, 25.56. LC-MS (m/z): 594.33 ([M+H]⁺). |
| (Iac) | ¹H-NMR (400 MHz, CDCl₃) δ (ppm): 8.36 (t, *J* = 7.4 Hz, 1.66H), 8.22 (s, 1H), 8.18 (s, 1.66H), 8.15 (s, 0.66H), 7.70 (t, *J* = 7.2 Hz, 1.66H), 7.59 (d, *J* = 7.8 Hz, 1.66H), 7.39 (t, *J* = 7.7 Hz, 2.33H), 7.33-7.27 (m, 2H), 7.25-7.19 (m, 2.33H), 7.19-7.14 (m, 2.33H), 7.14-7.08 (m, 2H), 6.95-6.82 (m, 4.32H), 6.72 (t, *J* = 7.8 Hz, 2H), 6.59 (d, *J* = 8.6 Hz, 1H), 4.94 (d, *J* = 10.5 Hz, 1H), 4.83 (s, 1H), 4.67 (s, 1H), 4.54-4.44 (m, 2H), 3.91 (s, 2H), 3.88 (s, 3H), 3.36 (ddd, *J* = 27.3, 15.2, 5.8 Hz, 2H), 3.21 (dd, *J* = 14.9, 8.7 Hz, 2H), 3.01 (s, 4H), 3.00 (s, 6H), 1.51 (s, 9H), 1.42 (s, 6H). ¹³C-NMR (100 MHz, CDCl₃) δ (ppm): 169.00, 168.71, 155.53, 151.14, 147.43, 147.05, 141.18, 136.50, 133.95, 133.59, 131.63, 130.51, 130.46, 129.62, 129.58, 127.41, 127.24, 123.14, 122.74, 122.63, 120.19, 120.09, 118.96, 118.79, 117.96, 117.59, 115.39, 112.69, 112.44, 112.38, 111.48, 111.42, 111.04, 110.24, 110.12, 80.76, 77.36, 52.65, 52.10, 51.96, 48.58, 48.46, 40.92, 28.60, 28.47, 28.38. LC-MS (m/z): 568.24 ([M+H]⁺). |
| (Iad) | ¹H-NMR (400 MHz, CDCl₃) δ (ppm): 8.46 (d, *J* = 8.3 Hz, 1H), 8.42 (d, *J* = 7.9 Hz, 0.5H), 8.21-8.12 (m, 3H), 7.74-7.68 (m, 1H), 7.66 (dd, *J* = 8.4, 2.3 Hz, 1H), 7.59 (s, 0.5H), 7.57 (d, *J* = 1.0 Hz, 0.5H), 7.56 (s, 0.5H), 7.53 (s, 0.5H), 7.47 (t, *J* = 1.6 Hz, 1H), 7.45 (s, 0.5H), 7.43 (d, *J* = 1.8 Hz, 2H), 7.41 (d, *J* = 1.0 Hz, 1.5H), 7.39 (s, 1H), 7.39-7.36 (m, 1.5H), 7.34 (s, 1.5H), 7.25-7.23 (m, 1H), 7.23-7.21 (m, 1H), 7.21-7.12 (m, 4.5H), 7.10 (d, *J* = 7.0 Hz, 1.5H), 6.95 (d, *J* = 7.7 Hz, 0.5H), 6.59 (d, *J* = 9.0 Hz, 1H), 5.30 (s, 1.5H), 5.17 (s, 0.5H), 4.95 (d, *J* = 9.7 Hz, 1H), 4.84 (d, *J* = 7.3 Hz, 1H), 4.68 (s, 1H), 4.54-4.43 (m, 2H), 3.92 (s, 1.5H), 3.89 (s, 3H), 3.43-3.29 (m, 2H), 3.23 (d, *J* = 8.7 Hz, 1.5H), 3.19 (d, *J* = 8.8 Hz, 1H), 1.52 (s, 9H), 1.42 (s, 4.5H). ¹³C-NMR (100 MHz, CDCl₃) δ (ppm): 168.73, 168.44, 155.57, 149.90, 148.00, 147.62, 142.23, 136.53, 134.09, 133.58, 133.20, 132.10, 130.45, 130.40, 130.27, 130.22, 129.94, 129.60, 128.87, 127.66, 127.38, 127.19, 126.11, 125.63, 124.83, 123.69, 123.15, 122.81, 122.70, 121.94, 120.24, 120.13, 119.38, 119.15, 119.07, 118.94, 118.75, 117.78, 117.39, 112.86, 112.73, 112.66, 112.50, 111.53, 111.46, 110.18, 110.03, 80.90, 77.36, 53.56, 52.59, 52.22, 52.08, 48.52, 48.44, 28.71, 28.46, 28.37, 26.29. LC-MS (m/z): 631.29 ([M+Na]⁺). |
| (Iae) | ¹H-NMR (400 MHz, CDCl₃) δ (ppm): 8.38 (t, *J* = 2.8 Hz, 1H), 8.37-8.30 (m, 3H), 8.27 (s, 1H), 8.23 (s, 1H), 8.12 (d, *J* = 2.3 Hz, 1H), 8.09 (d, *J* = 2.4 Hz, 1H), 7.71 (d, *J* = 9.3 Hz, 1H), 7.67 (dd, *J* = 2.7, 1.6 Hz, 1H), 7.65 (dt, *J* = 3.8, 1.8 Hz, 1H), 7.61 (dd, *J* = 6.6, 2.4 Hz, 1H), 7.58 (d, *J* = 2.6 Hz, 1H), 7.49 (s, 1H), 7.48-7.45 (m, 1H), 7.39 (t, *J* = 7.9 Hz, 2H), 7.25-7.18 (m, 2H), 7.17 (d, *J* = 2.8 Hz, 1H), 7.15 (s, 1H), 7.14-7.12 (m, 1H), 7.10 (d, *J* = 2.9 Hz, 1H), 6.91 (d, *J* = 8.6 Hz, 1H), 6.73 (d, *J* = 8.4 Hz, 1H), 6.72-6.66 (m, 2H), 6.58 (t, *J* = 9.2 Hz, 1H), 5.75 (s, 1H), 4.94 (d, *J* = 10.1 Hz, 1H), 4.82 (s, 1H), 4.67 (s, 1H), 4.53-4.41 (m, 2H), 3.89 (d, *J* = 2.8 Hz, 3H), 3.87 (s, 3H), 3.65 (s, 10H), 3.43-3.28 (m, 2H), 3.20 (dd, *J* = 14.9, 8.6 Hz, 2H), 2.82 (s, 3H), 2.71 (s, 10H), 1.51 (s, 9H), 1.41 (s, 9H), 1.32 (s, 2H), 1.15 (d, *J* = 2.6 Hz, 6H), 1.13 (d, *J* = 2.6 Hz, 6H). ¹³C-NMR (100 MHz, CDCl₃) δ (ppm): 168.83, 168.63, 168.53, 158.42, 155.53, 155.33, 149.50, 147.15, 146.75, 145.68, 145.56, 143.70, 136.50, 135.70, 135.63, 132.88, 132.49, 132.25, 129.47, 128.77, 127.78, 127.19, 126.71, 125.54, 123.15, 122.75, 122.63, 120.18, 120.07, 118.96, 118.78, 117.93, 117.52, 112.93, 112.72, 111.50, 111.43, 111.20, 110.18, 110.03, 107.05, 105.87, 83.60, 80.78, 77.36, 55.16, 52.55, 52.14, 52.00, 51.79, 48.53, 48.18, 45.42, 28.65, 28.46, 28.37, 24.94, 18.48. LC-MS (m/z): 652.40 ([M+H]⁺). |
| (Iaf) | ¹H-NMR (400 MHz, CDCl₃) δ (ppm): 8.68 (s, 1.5H), 8.65 (d, *J* = 7.3 Hz, 3H), 8.47 (d, *J* = 8.3 Hz, 1H), 8.43 (d, *J* = 8.1 Hz, 0.5H), 8.24 (s, 1H), 8.20 (s, 0.5H), 8.12 (d, *J* = 2.3 Hz, 0.5H), 8.09 (d, *J* = 2.4 Hz, 1H), 7.71 (d, *J* = 7.6 Hz, 1H), 7.61-7.55 (m, 1.5H), 7.45 (d, *J* = 9.0 Hz, 1H), 7.43-7.36 (m, 1.5H), 7.25-7.08 (m, 6H), 6.99 (d, *J* = 6.8 Hz, 0.5H), 6.59 (d, *J* = 8.7 Hz, 1H), 4.94 (d, *J* = 9.4 Hz, 1H), 4.84 (s, 1H), 4.68 (s, 1H), 4.48 (qd, *J* = 7.1, 5.6 Hz, 7H), 3.91 (s, 1.5H), 3.89 (s, 3H), 3.43-3.28 (m, 2H), 3.20 (dd, *J* = 14.7, 8.9 Hz, 2H), 1.51 (s, 9H), 1.49-1.44 (m, 7.5H), 1.42 (s, 4.5H), 1.26 (s, 1H), 0.91-0.86 (m, 1H). ¹³C-NMR (100 MHz, CDCl₃) δ (ppm): 168.46, 168.18, 165.30, 164.11, 157.03, 156.73, 155.55, 148.10, 147.72, 136.53, 132.92, 132.54, 129.88, 127.39, 127.13, 123.43, 123.17, 122.81, 122.69, 122.31, 120.23, 120.10, 118.93, 118.71, 117.68, 117.28, 113.11, 113.03, 111.55, 111.47, 110.13, 109.94, 80.90, 77.36, 64.15, 63.99, 52.49, 52.28, 52.15, 48.48, 48.39, 31.73, 28.78, 28.45, 28.37, 22.79, 14.61, 14.56, 14.26. LC-MS (m/z): 571.23 ([M+H]⁺). |
| (Iag) | ¹H-NMR (400 MHz, CDCl₃) δ (ppm): 8.36 (t, *J* = 8.5 Hz, 1.5H), 8.24-8.13 (m, 3H), 7.75-7.65 (m, 2H), 7.58 (t, *J* = 8.8 Hz, 2H), 7.50-7.31 (m, 6H), 7.22 (q, *J* = 7.8, 7.2 Hz, 6H), 7.18-7.07 (m, 3H), 6.91 (d, *J* = 8.1 Hz, 0.5H), 6.59 (d, *J* = 8.6 Hz, 1H), 4.95 (d, *J* = 9.7 Hz, 1H), 4.88-4.79 (m, 1H), 4.73-4.62 (m, 1H), 4.50 (dq, *J* = 12.5, 4.9, 4.5 Hz, 2H), 3.91 (s, 2H), 3.88 (s, 3H), 3.36 (ddd, *J* = 26.5, 14.9, 5.4 Hz, 2H), 3.21 (dd, *J* = 14.6, 8.4 Hz, 2H), 2.65 (q, *J* = 7.6, 6.9 Hz, 4H), 1.63 (dq, *J* = 15.2, 7.8, 6.9 Hz, 5H), 1.52 (s, 9H), 1.42 (s, 6H), 1.41-1.32 (m, 5H), 0.95 (td, *J* = 7.3, 3.6 Hz, 6H). ¹³C-NMR (100 MHz, CDCl₃) δ (ppm): 168.96, 168.66, 155.54, 147.30, 146.91, 141.77, 141.71, 137.41, 136.50, 135.84, 133.54, 133.18, 130.67, 130.60, 130.16, 130.11, 129.02, 128.98, 128.29, 127.40, 127.22, 126.36, 123.15, 122.72, 122.61, 120.17, 120.06, 118.93, 118.76, 117.96, 117.57, 112.77, 112.58, 112.50, 111.49, 111.43, 110.20, 110.06, 80.78, 77.36, 52.66, 52.09, 51.95, 48.57, 48.46, 35.38, 33.80, 28.61, 28.47, 28.38, 22.52, 22.51, 14.11. LC-MS (m/z): 604.31 ([M+Na]⁺). |
| (Iah) | ¹H-NMR (400 MHz, CDCl₃) δ (ppm): 8.37 (t, *J* = 8.7 Hz, 1.66H), 8.19 (d, *J* = 2.3 Hz, 0.66H), 8.18-8.14 (m, 2H), 8.13-8.10 (m, 0.66H), 7.71 (d, *J* = 8.1 Hz, 1H), 7.66 (dd, *J* = 8.7, 2.3 Hz, 0.66H), 7.58 (d, *J* = 7.9 Hz, 1H), 7.55 (dd, *J* = 8.6, 2.4 Hz, 1H), 7.42-7.40 (m, 0.66H), 7.39 (d, *J* = 2.1 Hz, 2.32H), 7.38-7.33 (m, 4H), 7.33-7.27 (m, 4.66H), 7.25-7.19 (m, 5.32H), 7.18 (d, *J* = 5.2 Hz, 1.66H), 7.16-7.14 (m, 1H), 7.12 (d, *J* = 1.8 Hz, 1H), 7.12-7.09 (m, 1.66H), 6.92 (d, *J* = 9.2 Hz, 0.66H), 6.57 (d, *J* = 8.6 Hz, 1H), 4.93 (d, *J* = 9.9 Hz, 1H), 4.82 (s, 1H), 4.72-4.62 (m, 1H), 4.53-4.42 (m, 2H), 4.05 (s, 1.33H), 4.04 (s, 2H), 3.91 (s, 2H), 3.88 (s, 3H), 3.43-3.28 (m, 1.66H), 3.22 (d, *J* = 8.4 Hz, 1H), 3.19 (d, *J* = 8.6 Hz, 0.66H), 1.58 (s, 3.32H), 1.51 (s, 9H), 1.45 (s, 1H), 1.42 (s, 6H), 1.35 (s, 1H), 1.26 (s, 0.66H), 0.83 (s, 1H). ¹³C-NMR (100 MHz, CDCl₃) δ (ppm): 168.93, 168.63, 155.53, 147.50, 147.12, 141.78, 141.73, 141.15, 140.28, 136.50, 133.77, 133.38, 130.55, 130.38, 130.32, 129.11, 129.06, 128.64, 127.66, 127.61, 127.21, 126.27, 124.39, 123.13, 122.78, 122.67, 120.22, 120.11, 118.98, 118.80, 117.91, 117.53, 112.76, 112.57, 112.49, 111.49, 111.42, 110.24, 110.11, 80.80, 77.36, 52.60, 52.13, 51.99, 48.53, 48.42, 42.15, 42.12, 28.64, 28.46, 28.38. LC-MS (m/z): 615.35 ([M+H]⁺). |
| (Iai) | ¹H-NMR (400 MHz, CDCl₃) δ (ppm): 8.37 (d, *J* = 8.0 Hz, 0.33H), 8.30 (d, *J* = 8.5 Hz, 1.33H), 8.23 (d, *J* = 7.6 Hz, 1.33H), 8.16 (s, 0.33H), 7.66 (ddd, *J* = 13.4, 7.4, 1.2 Hz, 0.33H), 7.60 (d, *J* = 8.1 Hz, 1H), 7.49 (d, *J* = 7.9 Hz, 0.33H), 7.45-7.39 (m, 0.33H), 7.34-7.23 (m, 7H), 7.23-7.14 (m, 4H), 7.11 (q, *J* = 7.5 Hz, 2H), 7.07-7.00 (m, 1.66H), 6.99 (s, 1.33H), 6.92 (s, 0.33H), 6.78-6.69 (m, 0.33H), 6.69-6.57 (m, 1.66H), 6.54 (d, *J* = 7.9 Hz, 0.33H), 6.47 (d, *J* = 8.2 Hz, 1H), 6.44-6.39 (m, 0.33H), 6.35 (s, 1H), 5.09 (s, 0.33H), 4.86 (d, *J* = 9.4 Hz, 1H), 4.48 (dp, *J* = 20.3, 6.6, 5.4 Hz, 4.66H), 4.37-4.28 (m, 1.33H), 3.33-3.23 (m, 0.33H), 3.19 (dd, *J* = 14.7, 6.1 Hz, 1H), 3.14-2.91 (m, 2H), 1.48 (s, 0.33H), 1.39 (s, 9H), 1.32 (s, 2.33H). ¹³C-NMR (100 MHz, CDCl₃) δ (ppm): 169.03, 155.52, 147.08, 146.70, 138.21, 136.48, 133.07, 129.26, 128.94, 128.90, 128.39, 127.98, 127.92, 127.77, 127.72, 127.55, 127.33, 123.27, 122.50, 122.39, 119.97, 119.84, 118.90, 117.78, 117.71, 117.13, 112.84, 111.47, 110.10, 80.57, 52.69, 48.39, 43.93, 43.86, 28.46, 28.37, 28.23, 28.12. LC-MS (m/z): 546.24 ([M+Na]⁺). |
| (Iaj) | ¹H-NMR (400 MHz, CDCl₃) δ (ppm): 8.20 (s, 1.5H), 7.67 (dd, *J* = 11.3, 8.3 Hz, 1.5H), 7.41 (dd, *J* = 13.5, 8.4 Hz, 3.5H), 7.26-7.20 (m, 2H), 7.20-7.10 (m, 5.5H), 6.72-6.61 (m, 2.5H), 6.47 (d, *J* = 8.0 Hz, 1H), 5.43 (s, 1H), 5.02 (d, *J* = 8.8 Hz, 1H), 4.85 (s, 0.5H), 4.70-4.58 (m, 1.5H), 4.51 (d, *J* = 11.5 Hz, 1.5H), 4.29 (s, 1.5H), 3.38 (dd, *J* = 14.5, 4.7 Hz, 0.5H), 3.31 (dd, *J* = 14.6, 5.3 Hz, 1.5H), 3.19 (d, *J* = 9.3 Hz, 1H), 3.16 (d, *J* = 9.3 Hz, 0.5H), 1.50 (s, 9H), 1.44 (s, 4.5H). ¹³C-NMR (100 MHz, CDCl₃) δ (ppm): 156.21, 155.74, 142.16, 142.06, 136.64, 136.51, 133.00, 132.93, 128.73, 128.56, 127.33, 127.05, 123.51, 123.12, 122.88, 120.36, 120.28, 120.18, 118.76, 118.10, 117.35, 112.08, 111.57, 110.52, 110.06, 109.54, 81.20, 77.36, 52.82, 52.06, 50.59, 50.20, 28.74, 28.46. LC-MS (m/z): 493.25 ([M+Na]⁺). |
| (Iak) | ¹H-NMR (400 MHz, CDCl₃) δ (ppm): 8.28 (d, *J* = 7.0 Hz, 1.3H), 8.18 (s, 1H), 8.14 (s, 0.3H), 7.91-7.82 (m, 1.3H), 7.61 (d, *J* = 7.7 Hz, 1H), 7.49 (d, *J* = 7.9 Hz, 0.3H), 7.36-7.20 (m, 3H), 7.16-7.09 (m, 2H), 7.08-6.99 (m, 3H), 6.97 (s, 0.3H), 6.78-6.61 (m, 2H), 6.42 (d, *J* = 8.3 Hz, 1H), 4.86 (d, *J* = 9.7 Hz, 1H), 4.72 (d, *J* = 23.7 Hz, 1H), 4.61-4.50 (m, 1H), 4.42-4.28 (m, 2H), 3.79 (s, 1H), 3.76 (s, 3H), 3.25 (ddd, *J* = 27.9, 14.8, 5.5 Hz, 1H), 3.11 (dd, *J* = 14.6, 8.5 Hz, 2H), 1.42 (s, 9H), 1.33 (s, 3H). ¹³C-NMR (100 MHz, CDCl₃) δ (ppm): 168.95, 168.65, 155.51, 148.28, 147.88, 136.48, 134.77, 131.96, 127.20, 123.13, 122.70, 122.60, 120.14, 120.04, 118.94, 118.92, 117.58, 117.51, 112.47, 111.97, 111.47, 110.19, 110.05, 80.74, 77.36, 52.58, 52.03, 51.88, 48.41, 48.27, 28.61, 28.45, 28.36. LC-MS (m/z): 471.23 ([M+Na]⁺). |
| (Ial) | ¹H-NMR (400 MHz, CDCl₃) δ (ppm): 9.41 (dd, *J* = 15.4, 8.1 Hz, 1.3H), 8.31 (s, 1H), 8.27 (s, 0.33H), 7.84-7.76 (m, 1.33H), 7.68 (d, *J* = 7.7 Hz, 1H), 7.56 (d, *J* = 7.9 Hz, 0.33H), 7.46-7.40 (m, 0.33H), 7.40-7.28 (m, 3H), 7.25-7.18 (m, 1.6H), 7.18-7.00 (m, 4H), 6.86 (d, *J* = 7.8 Hz, 0.33H), 6.77 (dt, *J* = 15.7, 7.6 Hz, 2H), 6.54 (d, *J* = 8.4 Hz, 1H), 4.94 (d, *J* = 9.5 Hz, 1H), 4.89 (d, *J* = 5.9 Hz, 0.33H), 4.83 (dd, *J* = 8.7, 4.9 Hz, 0.33H), 4.66-4.55 (m, 1H), 4.46 (ddt, *J* = 13.4, 8.1, 4.8 Hz, 1.6H), 3.33 (ddd, *J* = 26.5, 14.9, 5.6 Hz, 1.6H), 3.19 (dd, *J* = 14.5, 8.1 Hz, 2H), 2.61 (s, 1H), 2.58 (s, 3H), 1.50 (s, 9H), 1.41 (s, 3H). ¹³C-NMR (100 MHz, CDCl₃) δ (ppm): 201.73, 201.33, 155.51, 148.24, 147.86, 136.47, 135.65, 135.23, 132.89, 127.21, 123.21, 122.59, 122.47, 120.05, 119.92, 119.54, 118.87, 117.82, 117.51, 117.11, 117.06, 112.20, 111.49, 111.44, 110.02, 109.95, 80.72, 77.16, 52.56, 47.90, 28.45, 28.35, 28.29, 28.16. LC-MS (m/z): 455.19 ([M+Na]⁺). |
| (Iam) | ¹H-NMR (400 MHz, CDCl₃) δ (ppm): 8.29 (s, 1.5H), 7.76 (dd, *J* = 8.8, 2.7 Hz, 3.5H), 7.56 (t, *J* = 7.7 Hz, 1.5H), 7.31 (d, *J* = 8.1 Hz, 1.5H), 7.19-7.11 (m, 2H), 7.11-7.00 (m, 3.5H), 6.41 (d, *J* = 8.7 Hz, 3H), 5.59 (s, 1H), 5.21-5.10 (m, 0.5H), 5.02 (d, *J* = 8.1 Hz, 1H), 4.94 (s, 0.5H), 4.43 (d, *J* = 8.4 Hz, 1H), 4.35 (dd, *J* = 13.4, 5.7 Hz, 1H), 4.23 (s, 1H), 3.77 (s, 1.5H), 3.76 (s, 3H), 3.32-3.13 (m, 2.5H), 3.06 (dd, *J* = 14.7, 9.3 Hz, 1H), 1.97 (s, 0.5H), 1.91 (s, 1H), 1.40 (s, 9H), 1.34 (s, 4.5H). ¹³C-NMR (100 MHz, CDCl₃) δ (ppm): 167.22, 157.01, 149.11, 148.77, 136.49, 131.67, 131.60, 126.93, 123.59, 123.27, 122.80, 120.22, 118.54, 118.08, 117.11, 112.48, 111.69, 109.69, 81.45, 53.26, 51.87, 50.71, 28.55, 28.34, 28.32, 2.02, 1.13. LC-MS (m/z): 471.33 ([M+Na]⁺). |

### Example 2: Anti-viral effect of compounds of formula (I)

### Measurement of protective effect against virus-induced cytopathic effect:

Vero-E6 cells were seeded onto 96-well plates (1×10⁴ cells/well). Candidate compounds were diluted from stock solutions in complete media (DMEM) supplemented with 10 mM HEPES, 1X non-essential amino acids (Gibco), 100 U/mL penicillin-streptomycin (Gibco) and 2% fetal bovine serum (heat-inactivated at 56 °C for 30 min)) to achieve the desired concentration. A wide range of compound concentrations from 50 to 0.78 µM were tested in an initial experiment.

SARS-CoV-2 stock strain NL/2020 (provided by Dr. R. Molenkamp, Erasmus University Medical Center Rotterdam) was diluted to achieve a multiplicity of infection (MOI) of 0.001. Virus-compound mixture was used to inoculate the Vero-E6 monolayer. Cultures were maintained at 37 °C in a 5% CO₂ incubator for 72 h in a BSL3 facility. Cells were fixed using a 4% formaldehyde-PBS solution to finalize the experiment and inactivate the virus. Virus-induced cell death was revealed by staining with a 0.1% crystal violet solution in water-methanol for 30-60 minutes, time after which, plates were extensively washed with water and dried. Infected, as well as uninfected cells, were used as controls. Using crystal violet staining, the dilutions of compounds capable of protecting the cell monolayer from virus-induced cell death were determined. Concentration of the compound present at the lowest dilutions protecting 100% of the cell monolayer are averaged with the immediate lower dilution to estimate an effective concentration 50 (EC₅₀). The ratio between the highest and the lowest protective concentration (PC max and PC min) is used to estimate a preliminary therapeutic index, as it corresponds to the range of concentrations that are effective and non-cytotoxic to the cell monolayer. These procedures were used in the Example 3 and Figure 1. Table 3 provides an estimate EC₅₀ value according to the method described above for the compounds of Table 1.

**Table3**

| Compound number | PC MAX (µM) | PC MIN (µM) | ESTIMATED EC₅₀ (µM) |
|---|---|---|---|
| (Ia) | 25.00 | 0.78 | 0.58 |
| (Ib) | 12.50 | 0.78 | 0.58 |
| (Ic) | 50.00 | 0.78 | 0.58 |
| (Id) | 25.00 | 0.78 | 0.58 |
| (Ie) | 12.50 | 0.78 | 0.58 |
| (If) | 50.00 | 0.78 | 0.58 |
| (Ig) | 50.00 | 0.78 | 0.58 |
| (Ih) | 12.50 | 1.56 | 1.17 |
| (Ii) | 12.50 | 1.56 | 1.17 |
| (Ij) | 25.00 | 1.56 | 1.17 |
| (Ik) | 12.50 | 1.56 | 1.17 |
| (Il) | 25.00 | 1.56 | 1.17 |
| (Im1) | 25.00 | 1.56 | 1.17 |
| (Im2) | 50.00 | 1.56 | 1.17 |
| (In) | 3.12 | 1.56 | 1.17 |
| (Io) | 50.00 | 1.56 | 1.17 |
| (Ip) | 50.00 | 1.56 | 1.17 |
| (Iq) | 25.00 | 1.56 | 1.17 |
| (Ir) | 3.12 | 1.56 | 1.17 |
| (Is) | 50.00 | 1.56 | 1.17 |
| (It) | 50.00 | 1.56 | 1.17 |
| (Iu) | 12.50 | 3.12 | 2.34 |
| (Iv) | 12.50 | 3.12 | 2.34 |
| (Iw) | 25.00 | 3.12 | 2.34 |
| (Ix) | 25.00 | 3.12 | 2.34 |
| (Im3) | 50.00 | 3.12 | 2.34 |
| (Iy) | 50.00 | 3.12 | 2.34 |
| (Iz) | 12.50 | 3.12 | 2.34 |
| (laa) | 12.50 | 3.12 | 2.34 |
| (lab) | 50.00 | 3.12 | 2.34 |
| (lac) | 50.00 | 3.12 | 2.34 |
| (lad) | 25.00 | 3.12 | 2.34 |
| (lae) | 3.12 | 3.12 | 2.34 |
| (Iaf) | 50.00 | 3.12 | 2.34 |
| (Iag) | 25.00 | 6.25 | 4.68 |
| (Iah) | 50.00 | 6.25 | 4.68 |
| (Iai) | 25.00 | 6.25 | 4.69 |
| (Im4) | 50.00 | 6.25 | 4.69 |
| (Iaj) | 12.50 | 6.25 | 4.69 |
| (Iak) | 12.50 | 12.50 | 9.37 |
| (Ial) | 25.00 | 12.50 | 9.37 |
| (Iam) | 25.00 | 25.00 | 18.75 |

### Measurement of intracellular viral antigen accumulation:

To confirm that the monolayer protection effect displayed by the compounds of the invention is directly related to an antiviral effect, Vero-E6, A549-ACE2 or Calu3 cell monolayers were inoculated at low multiplicity with SARS-CoV-2 stock strain NL/2020 in the presence of non-toxic compound concentrations. Twenty-four hours later (48 hours for A549-ACE2 and Calu3) cells were fixed by 20 minutes incubation with a 4% formaldehyde-PBS solution. After extensive washes with PBS, infection efficiency was evaluated by the detection of intracellular SARS-CoV-2 N protein accumulation by immunofluorescence microscopy. Nuclei were stained using DAPI (4',6-diamidino-2-phenylindole) dye. Automated imaging was carried out in a SparkCyto (Tecan) multimode plate reader. The total intensity signal present in vehicle-treated conditions was used as reference (100%). This procedure was used in the Example 4 and Table 4.

### Determination of cytotoxicity by MTT colorimetric assay:

To evaluate the cytotoxicity profile of the selected compounds MTT assays were performed in Vero-E6, A549-ACE2 and Calu3 cells. Uninfected cells were incubated with a range of compound for forty-eight hours, time at which the MTT substrate [3-(4,5-dimethylthiazol-2-yl)-2,5-diphenyltetrazolium bromide] was added and the formation of formazan precipitates was evaluated two hours later, using a colorimetric readout. Vehicle-treated cells were used as reference (100%). This procedure was used in the Example 4 and Table 4.

### Intracellular viral RNA quantification:

To evaluate the full potential of the compounds of formula (I) in cell culture, A549-ACE2 cell monolayers were inoculated at MOI 0.005 with SARS-CoV-2 stock strain NL/2020 in the presence of non-toxic compound concentrations. Forty-eight hours later cell lysates were prepared using Trizol reagent (Thermo Scientific) and the viral RNA content was determined by RT-qPCR using previously validated sets of primers and probes specific for the detection of the SARS-CoV-2 E gene and the cellular 28S gene, for normalization purposes. ΔCt method was used for relative quantitation of the intracellular viral RNA accumulation in compound-treated cells compared to the levels in infected cells treated with DMSO, set as 100%. This procedure was used in the Example 5 and Table 5.

### Activity on recombinant human coronavirus 229E-GFP

Huh7 cells (provided by Dr. Chisari, TSRI-La Jolla, CA) were maintained subconfluent in complete media [(DMEM supplemented with 10 mM HEPES, 1X non-essential amino acids (Gibco), 100 U/ mL penicillin-streptomycin (Gibco) and 10 % Fetal Bovine Serum (FBS; heat-inactivated at 56 °C for 30 min)]. Huh7 cells were seeded onto 96-well plates (1×10⁴ cells/well). After 24 hours, stock solutions of the compound of formula (I) (10 mM in DMSO) were serially diluted into complete cell culture media to achieve the desired 4X concentration. On the other hand, hCoV-229E-GFP virus (Human coronavirus 229E expressing Green Fluorescent Protein) stock (provided by Dr. Volker Thiel; University of Bern) was diluted in complete media to achieve a final MOI of 0.01. One hundred microliters (100 µl) of the virus dilution were mixed 1:1 with 100 µL of the compound dilution to achieve the desired final compound concentrations. 100 µL of the mixture was applied onto the Huh7 cell monolayer in biological replicates and cells were cultured for 48 hours at 33 °C in a 5% CO₂ incubator. Cells were fixed in a 4% formaldehyde solution in PBS for 10 minutes at room temperature, washed twice with PBS and nuclei were stained using DAPI (4',6-diamidino-2-phenylindole) dye to reveal cell nuclei individual well fluorescence was measured in a Spark Cyto (Tecan) device. Background subtraction was performed using non-infected wells and signal was normalized to the average fluorescence found in vehicle (DMSO)-treated virus-infected wells in the green (GFP) channel for infection efficiency and in the blue (DAPI) channel to estimate cell biomass. This procedure was used in the Example 6 and Figure 2.

### Activity on recombinant human coronavirus West Nile virus (WNV-GFP)

Huh7 cells were seeded onto 96-well plates (1×10⁴ cells/well). After 24 hours, stock solutions of the compound of formula (I) (10 mM in DMSO) were serially diluted into complete cell culture media to achieve the desired 4X concentration. On the other hand, WNV-GFP virus (dicistronic, recombinant West Nile Virus expressing Green Fluorescent Protein) stock (provided by Robert Doms (University of Pennsylvania; Philadelphia, PA) was diluted in complete media to achieve a final MOI of 0.01. 100 µL of the virus dilution were mixed 1:1 with 100 µL of the compound dilution to achieve the desired final compound concentrations. 100 µL of the mixture was applied onto the Huh7 cell monolayer in biological replicates and cells were cultured for 48 hours at 37 °C in a 5% CO₂ incubator in a BSL3 facility. Cells were fixed in a 4% formaldehyde solution in PBS for 10 minutes at room temperature, washed twice with PBS and nuclei were stained using DAPI (4',6-diamidino-2-phenylindole) dye to reveal cell nuclei individual well fluorescence was measured in a Spark Cyto (Tecan) device. Background subtraction was performed using non-infected wells and signal was normalized to the average fluorescence found in vehicle (DMSO)-treated virus-infected wells in the green (GFP) channel for infection efficiency and in the blue (DAPI) channel to estimate cell biomass. This procedure was used in the Example 6 and Figure 2.

### Activity on recombinant vesicular stomatitis virus (VSV-GFP).

A549-ACE2 cells were seeded onto 96-well plates (2×10⁴ cells/well). After 24 hours, stock solutions of the compound of formula (I) (10 mM in DMSO) were serially diluted into complete cell culture media to achieve the desired 4X concentration. On the other hand, VSV-GFP virus (recombinant Vesicular Stomatitis Virus expressing Green Fluorescent Protein) stock (provided by Dolores Rodriguez (CNB-CSIC; Madrid, Spain) was diluted in complete media to achieve a final MOI of 0.01. 100 µL of the virus dilution were mixed 1:1 with 100 µL of the compound dilution to achieve the desired final compound concentrations. 100 µL of the mixture was applied onto the A549-ACE2 cell monolayer in biological replicates and cells were cultured for 18-20 hours at 37°C in a 5% CO₂ incubator. Cells were fixed in a 4% formaldehyde solution in PBS for 10 minutes at room temperature, washed twice with PBS and nuclei were stained using DAPI (4',6-diamidino-2-phenylindole) dye to reveal cell nuclei individual well fluorescence was measured in a Spark Cyto (Tecan) device. Background subtraction was performed using non-infected wells and signal was normalized to the average fluorescence found in vehicle (DMSO)-treated virus-infected wells in the green (GFP) channel for infection efficiency and in the blue (DAPI) channel to estimate cell biomass. This procedure was used in the Example 6 and Figure 2.

### Example 3: Study of protective activity of the compounds.

Vero-E6 cell cultures were inoculated with SARS-CoV-2 (MOI 0.001) in the presence of serial 2-fold dilutions (50-0.8 µM) of the indicated compounds of formula (Ic), (Ia), (Iq), (Id) and (Ie) in two biological replicates. Seventy-two hours later cells were fixed and stained with crystal violet to verify cell monolayer integrity. Controls include uninfected wells (mock-infected) and SARS-CoV-2 infected wells in the absence of compounds (0µM). Compounds displaying protective activity are identified as compounds for which positive staining, similar to mock-infected cells, is observed in cells inoculated with the virus. The results of this experiment are shown in Figure 1. This approach was used to study the protective activity of all the compounds of the invention (see Table 3).

### Example 4: Estimation of the antiviral potency indexes (EC₅₀ and EC₉₀) and cytotoxicity of selected compounds.

Vero-E6, A549-ACE2 and Calu3 cell lines were inoculated at MOI 0.005 in the presence of increasing concentrations of the selected compounds (see Table 4) and incubated to enable propagation in the well. In this experimental setup, viral antigen staining in the cells is proportional to the overall virus propagation efficiency. Based on viral antigen staining, dose-response curves were built to estimate the concentrations of compound reducing SARS-CoV-2 infection efficiency by 50% (EC₅₀) or 90% (EC₉₀) as compared with the vehicle control. Parallel uninfected cultures were treated with the same compound concentrations and overall cell viability was estimated using an MTT assay to verify that antiviral doses are not overtly cytotoxic. Cytotoxicity index (CC₅₀) was estimated as the compound dose required to reduce cell viability by 50% as compared with the vehicle control.

Table 4 shows the values of EC₅₀, EC₉₀ and MTT-CC₅₀ for certain compounds of formula (I) obtained in Vero-E6, A549-ACE2 and Calu3 cell lines.

**Table 4**

| | **Vero-E6 (µM)** | | | **A549-ACE2 (µM)** | | | **Calu-3 (µM)** | | |
|---|---|---|---|---|---|---|---|---|---|
| **Compound name** | **EC₅₀** | **EC₉₀** | **MTT CC₅₀** | **EC₅₀** | **EC₉₀** | **MTT CC₅₀** | **EC₅₀** | **EC₉₀** | **MTT CC₅₀** |
| (Ib) | 0.5 | 2.2 | >12.5 | 1.8 | 2.7 | 24 | 1.2 | 2.5 | >50 |
| (Ii) | 0.2 | 1.4 | >12.5 | 2.0 | 6.0 | 14 | <0.9 | 2.7 | 50 |
| (Ih) | 0.1 | 2.5 | >12.5 | 4.0 | 7.0 | 15 | 3.7 | 4.0 | >50 |
| (Id) | 0.2 | 0.5 | >50 | 0.6 | 1.4 | 15 | 0.3 | 1.1 | >50 |
| (Ip) | 1.5 | 7.0 | >50 | 5.0 | 9.0 | 15 | 1.8 | 8.0 | 50 |
| (Ia) | 0.3 | 0.5 | >50 | 1.0 | 1.8 | >50 | 0.2 | 2.0 | >50 |
| (Iq) | 0.2 | 0.9 | >50 | 1.5 | 5.0 | >50 | 0.3 | 2.5 | >50 |
| (Is) | 0.4 | 1.8 | >50 | 2.0 | 8.0 | >50 | 1.3 | 8.0 | >50 |
| (Ig) | 1.3 | 2.0 | >50 | 1.8 | 8.0 | >50 | 1.8 | 8.0 | >50 |
| (Io) | 1.3 | 2.0 | >50 | 4.5 | 9.0 | >50 | 5.0 | 8.1 | 50 |
| (Ic) | 0.2 | 0.3 | >50 | 1.0 | 2.1 | 14 | 1.2 | 1.8 | >50 |
| (If) | 1.0 | 1.8 | >50 | 1.0 | 1.8 | >50 | 2.0 | 4.0 | 50 |

### Example 5: Viral load reduction assessed by RT-qPCR.

Selected compounds were tested for their ability to control SARS-CoV-2 propagation by inoculating A549-ACE2 cells with SARS-COV-2 at MOI 0.005 in the presence of non-toxic compound concentrations, as indicated in the Table 5. Total RNA was extracted at 48 hours post-inoculation and relative viral RNA levels, normalized to 28S house-keeping gene, were calculated using ΔCt method. Data are shown as the average LOG reduction of three biological replicates.

**Table 5**

| **Compound** | **Compound concentration [µM]** | **LOG viral RNA reduction** |
|---|---|---|
| DMSO (vehicle) | 0 | 0 |
| (Ic) | 4 | 4.26 |
| (If) | 4 | 5.14 |
| Remdesivir | 5 | 6.67 |

The results of Table 5 show that the compounds of formulae (Ic) and (If) are effective in preventing propagation of SARS-CoV-2. Data for remdesivir are provided for comparison purposes.

### Example 6: Antiviral activity against hCoV-229GFP but not non-coronaviruses

Selected compounds were tested for their ability to interfere with a model of common cold coronavirus 229E (229E-GFP; MOI 0.01) in Huh-7 cell cultures. In parallel, the same cells were treated with the same doses of compound and inoculated with a recombinant West Nile virus expressing GFP (WNV-GFP; MOI 0.01). In parallel, A549-ACE2 cells were treated with the same doses of compound and inoculated with a recombinant vesicular stomatitis virus expressing GFP (VSV-GFP; MOI 0.01). Cell cultures were incubated at 37 °C (33 °C for 229E-GFP) until viral infection had propagated throughout the culture (18-20h for VSV-GFP; 48h for WNV-GFP or 229E-GFP). Cells were fixed and viral propagation was estimated as a function of GFP expression in the experimental well (Figure 2, panels A, C). Cell biomass was determined by DAPI staining to verify the absence of an overt cytotoxicity (Figure 2, panels B, D). The results of Figure 2 show that the compounds of formulae (Ia), (Ic) and (Iq) are suitable for preventing efficiently the infection with a virus of the coronavirus type such as 229E, while not being suitable for preventing the infection by Vesicular Stomatitis Virus or West Nile Virus.

## Claims

1. A compound of formula (I) or a pharmaceutically acceptable salt thereof 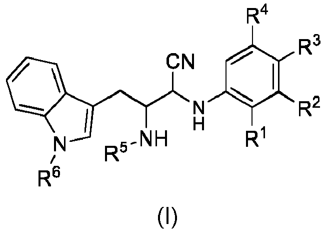 wherein R¹ is a group selected from the group consisting of hydrogen, halogen, carboxyl (-COOH), (C₁-C₄)alkyloxycarbonyl, (C₁-C₄)alkylcarbonyl, phenylcarbonyl, and a group of formula -CONH-(CH₂)ₙ-Ph wherein n is 0 or 1;
R² is a group selected from the group consisting of hydrogen, halogen, (C₁-C₄)alkyloxycarbonyl, and phenylcarbonyl;
R³ is a group selected from the group consisting of:
a) hydrogen,
b) halogen,
c) (C₁-C₄)alkyl,
d) (C₁-C₄)alkyloxycarbonyl, and
e) a group selected from the group consisting of pyridinyl, pyrimidinyl and phenyl, said group being optionally substituted at any available position with one or two groups selected from the group consisting of halogen, (C₁-C₄)alkyl, (C₁-C₄)alkyloxy, trilfuoromethyloxy, di((C₁-C₄)alkyl)amino, phenyl, benzyl, (C₃-C₆)cycloalkyl and a 5 to 6-membered saturated heterocyclic ring, the members of the ring being selected from the group consisting of C, CH, CH₂, O, N and NH, said ring being optionally substituted at any available position with a (C₁-C₄)alkyl; or, alternatively, wherein said group selected from the group consisting of pyridinyl, pyrimidinyl and phenyl optionally forms a 6-membered ring with a divalent group of formula -O-(CH₂)₂-O- whereby the oxygen atoms of said divalent group are connected to two adjacent carbon atoms of said pyridinyl, pyrimidinyl or phenyl group;
R⁴ is a group selected from the group consisting of hydrogen, halogen and phenyl optionally substituted at any available position with a (C₁-C₄)alkyl group;
R⁵ and R⁶ are each a group independently selected from the group consisting of hydrogen and a group of formula C₁-₄-alkyl-(O)ₓCO-, wherein x has a value of 0 or 1 and wherein the C₁₋₄-alkyl group is optionally substituted with 1 to 3 halogen groups;
with the proviso that the compound of formula (I) is not a compound of formula (11) or (12)

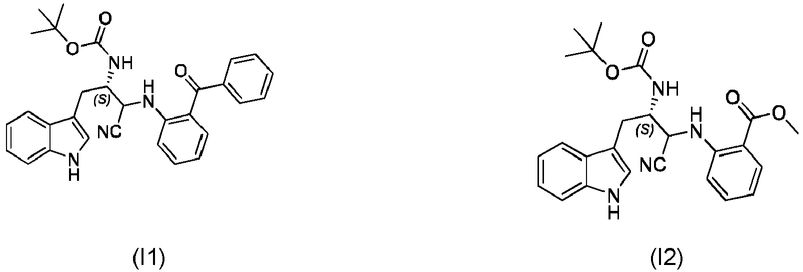

2. A compound of formula (I) as defined in claim 1 that is a compound of formula (I') or a pharmaceutically acceptable salt thereof

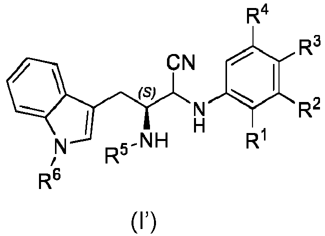

3. A compound of formula (I) as defined in any one of claims 1 to 2 or a pharmaceutically acceptable salt thereof wherein R¹ is a group selected from the group consisting of hydrogen, bromide, methyloxycarbonyl, ethyloxycarbonyl, *tert-*butyloxycarbonyl, methylcarbonyl, phenylcarbonyl, and a radical of formula -CONH-(CH₂)ₙ-Ph wherein n is 0 or 1.

4. A compound of formula (I) as defined in any one of claims 1 to 3 or a pharmaceutically acceptable salt thereof wherein R² is hydrogen or bromide.

5. A compound of formula (I) as defined in any one of claims 1 to 4 or a pharmaceutically acceptable salt thereof wherein R³ is a group selected from the group consisting of hydrogen, bromide, chloride, methyloxycarbonyl, 4-(*N*-morpholinyl)phenyl, 3-(*N*-morpholinyl)phenyl, 4-*tert*-butylphenyl, 4-ethylphenyl, 4-cyclopropylphenyl, 2,3-dihydro-benzo[1,4]dioxin-6-yl, 4-(4-methylpiperazin-1-yl)phenyl, 4-butylphenyl, 3-benzylphenyl, 2-fluoro-5-isopropyloxyphenyl, 6-pyrrodinylpyridin-3-yl, 6-methylpyridin-3-yl, 6-(*N*-morpholinyl)pyridine-3-yl, 6-methoxypyridin-3-yl, 6-ethoxypyrimidin-3-yl, 4-diethylaminophenyl, 4-propyloxyphenyl, 3-*tert*-butylphenyl, 3-ethylphenyl, 3-pyrrolidinylphenyl, 3-diethylaminophenyl, 3-trifluoromethoxyphenyl, and 6-(4-isopropylpiperazin-1-yl)pyridin-3-yl.

6. A compound of formula (I) as defined in any one of claims 1 to 5 or a pharmaceutically acceptable salt thereof wherein R⁴ is hydrogen.

7. A compound of formula (I) as defined in any one of claims 1 to 6 or a pharmaceutically acceptable salt thereof wherein R⁵ is *tert*-butyloxycarbonyl.

8. A compound of formula (I) as defined in any one of claims 1 to 7 or a pharmaceutically acceptable salt thereof wherein R⁶ is hydrogen.

9. A compound of formula (I) as defined in any one of claims 1 to 8 or a pharmaceutically acceptable salt thereof that is selected from the group consisting of:
methyl 4-(((2*S*)-2-((*tert*-butoxycarbonyl)amino)-1-cyano-3-(1*H*-indol-3-yl)propyl)-amino)-4'-morpholino-[1,1'-biphenyl]-3-carboxylate;
methyl 5-bromo-2-(((2*S*)-2-((*tert*-butoxycarbonyl)amino)-1-cyano-3-(1*H*-indol-3-yl)propyl)amino)benzoate;
(3S)-2-((4-Bromo-2-benzoylphenyl)amino)-4-(1*H*-indol-3-yl)-3-(*tert-*butoxycarbonylamino) butanenitrile;
(3S)-3-(*tert*-Butoxycarbonylamino)-4-(1*H*-indol-3-yl)-2-((2-benzoyl-4-chlorophenyl)amino) butanenitrile;
methyl 5-bromo-2-(((2*S*)-2-((*tert*-butoxycarbonyl)amino)-1-cyano-3-(1*H*-indol-3-yl)propyl)amino)benzoate;
methyl 2-(((2*S*)-2-((*tert*-butoxycarbonyl)amino)-1-cyano-3-(1*H*-indol-3-yl)propyl)amino)-5-(2,3-dihydrobenzo[*b*][1,4]dioxin-6-yl)benzoate;
methyl 4-(((2*S*)-2-((*tert*-butoxycarbonyl)amino)-1-cyano-3-(1*H*-indol-3-yl)propyl)amino)-3'-morpholino-[1,1'-biphenyl]-3-carboxylate;
ethyl 2-(((2*S*)-2-((*tert*-butoxycarbonyl)amino)-1-cyano-3-(1*H*-indol-3-yl)propyl)amino)-benzoate;
methyl 4-bromo-2-(((2*S*)-2-((*tert*-butoxycarbonyl)amino)-1-cyano-3-(1*H*-indol-3-yl)propyl)-amino)benzoate;
methyl 4-(((2*S*)-2-((*tert*-butoxycarbonyl)amino)-1-cyano-3-(1*H*-indol-3-yl)propyl)amino)-4'-(*tert*-butyl)-[1,1'-biphenyl]-3-carboxylate;
methyl 2-bromo-6-(((2*S*)-2-((*tert*-butoxycarbonyl)amino)-1-cyano-3-(1*H*-indol-3-yl)propyl)-amino)benzoate;
methyl 4-(((2*S*)-2-((*tert*-butoxycarbonyl)amino)-1-cyano-3-(1*H*-indol-3-yl)propyl)amino)-4'-ethyl-[1,1'-biphenyl]-3-carboxylate;
methyl 4-(((2*S*)-2-((*tert*-butoxycarbonyl)amino)-1-cyano-3-(1*H*-indol-3-yl)propyl)amino)-4'-cyclopropyl-[1,1'-biphenyl]-3-carboxylate;
methyl 4-(((2*S*)-2-((*tert*-butoxycarbonyl)amino)-1-cyano-3-(1*H*-indol-3-yl)propyl)amino)-4'-(4-methylpiperazin-1-yl)-[1,1'-biphenyl]-3-carboxylate;
methyl 4-(((2*S*)-2-((*tert*-butoxycarbonyl)amino)-1-cyano-3-(1*H*-indol-3-yl)propyl)amino)-2'-fluoro-5'-isopropoxy-[1,1'-biphenyl]-3-carboxylate;
methyl 2-(((2*S*)-2-((*tert*-butoxycarbonyl)amino)-1-cyano-3-(1*H*-indol-3-yl)propyl)amino)-5-(6-(pyrrolidin-1-yl)pyridin-3-yl)benzoate;
methyl 2-(((2*S*)-2-((*tert*-butoxycarbonyl)amino)-1-cyano-3-(1*H*-indol-3-yl)propyl)amino)-5-(6-methylpyridin-3-yl)benzoate;
methyl 2-(((2*S*)-2-((*tert*-butoxycarbonyl)amino)-1-cyano-3-(1*H*-indol-3-yl)propyl)amino)-5-(6-morpholinopyridin-3-yl)benzoate;
methyl 2-(((2*S*)-2-((*tert*-butoxycarbonyl)amino)-1-cyano-3-(1*H*-indol-3-yl)propyl)amino)-5-(6-methoxypyridin-3-yl)benzoate;
methyl 2-(((2*S*)-2-((*tert*-butoxycarbonyl)amino)-1-cyano-3-(1*H*-indol-3-yl)propyl)amino) benzoate;
*tert*-Butyl2-(((2*S*)-2-((*tert*-butoxycarbonyl)amino)-1-cyano-3-(1*H*-indol-3-yl)propyl)amino)-benzoate;
methyl 4-(((2*S*)-2-((*tert*-butoxycarbonyl)amino)-1-cyano-3-(1*H*-indol-3-yl)propyl)amino)-4'-butyl-[1,1'-biphenyl]-3-carboxylate;
methyl 4-(((2*S*)-2-((*tert*-butoxycarbonyl)amino)-1-cyano-3-(1*H*-indol-3-yl)propyl)amino)-4'-(diethylamino)-[1,1'-biphenyl]-3-carboxylate;
methyl 4-(((2S)-2-((tert-butoxycarbonyl)amino)-1-cyano-3-(1H-indol-3-yl)propyl)amino)-4'-(cyclopropyl)-[1,1'-biphenyl]-3-carboxylate;
methyl 4-(((2*S*)-2-((*tert*-butoxycarbonyl)amino)-1-cyano-3-(1*H*-indol-3-yl)propyl)amino)-4'-propoxy-[1,1'-biphenyl]-3-carboxylate;
methyl 4-(((2*S*)-2-((*tert*-butoxycarbonyl)amino)-1-cyano-3-(1*H*-indol-3-yl)propyl)amino)-3'-(tert-butyl)-[1,1'-biphenyl]-3-carboxylate;
methyl 4-(((2*S*)-2-((*tert*-butoxycarbonyl)amino)-1-cyano-3-(1*H*-indol-3-yl)propyl)amino)-3'-ethyl-[1,1'-biphenyl]-3-carboxylate;
methyl 4-(((2*S*)-2-((*tert*-butoxycarbonyl)amino)-1-cyano-3-(1*H*-indol-3-yl)propyl)amino)-3'-(pyrrolidin-1-yl)-[1,1'-biphenyl]-3-carboxylate;
methyl 4-(((2*S*)-2-((*tert*-butoxycarbonyl)amino)-1-cyano-3-(1*H*-indol-3-yl)propyl)amino)-3'-(dimethylamino)-[1,1'-biphenyl]-3-carboxylate;
methyl 4-(((2*S*)-2-((*tert*-butoxycarbonyl)amino)-1-cyano-3-(1*H*-indol-3-yl)propyl)amino)-3'-(trifluoromethoxy)-[1,1'-biphenyl]-3-carboxylate;
methyl 2-(((2*S*)-2-((*tert*-butoxycarbonyl)amino)-1-cyano-3-(1*H*-indol-3-yl)propyl)amino)-5-(6-(4-isopropylpiperazin-1-yl)pyridin-3-yl)benzoate;
methyl 2-(((2*S*)-2-((*tert*-butoxycarbonyl)amino)-1-cyano-3-(1*H*-indol-3-yl)propyl)amino)-5-(2-ethoxypyrimidin-5-yl)benzoate;
methyl 4-(((2*S*)-2-((*tert*-butoxycarbonyl)amino)-1-cyano-3-(1*H*-indol-3-yl)propyl)amino)-4'-butyl-[1,1'-biphenyl]-3-carboxylate;
methyl 3'-benzyl-4-(((2*S*)-2-((*tert*-butoxycarbonyl)amino)-1-cyano-3-(1*H*-indol-3-yl)propyl)-amino)-[1,1'-biphenyl]-3-carboxylate;
2-(((2*S*)-2-(*tert*-butoxycarbonylamino)-1-cyano-3-(1*H*-indol-3-yl)propyl)amino)-*N-*benzyl-benzamide;
(3*S*)-2-((2-bromophenyl)amino)-4-(1*H*-indol-3-yl)-3-(*tert*-butoxycarbonylamino) butanenitrile;
methyl 2-(((2*R*)-2-((*tert*-butoxycarbonyl)amino)-1-cyano-3-(1*H*-indol-3-yl)propyl)amino)-benzoate;
(3*S*)-3-(*tert*-butoxycarbonylamino)-2-((2-acetylphenyl)amino)-4-(1*H*-indol-3-yl)butanenitrile; and
methyl 4-(((2*S*)-2-((*tert*-butoxycarbonyl)amino)-1-cyano-3-(1*H*-indol-3-yl)propyl)amino)-benzoate.

10. A composition comprising a compound of formula (I) or a compound of formula (11) or a compound of formula (12) as defined in any of claims 1 to 9, or a pharmaceutically acceptable salt thereof and one or more pharmaceutically acceptable diluent or carrier.

11. A compound of formula (I) or a compound of formula (11) or a compound of formula (12) as defined in any of claims 1 to 9, or a pharmaceutically acceptable salt thereof, or a composition as defined in claim 10 for use in medicine.

12. A compound of formula (I) or a compound of formula (I1) or a compound of formula (I2), as defined in any of claims 1 to 9 or a pharmaceutically acceptable salt thereof, or a composition as defined in claim 10 for use in the treatment or prevention of viral infection by coronavirus.

13. Process for the preparation of a compound of formula (I) as defined in any of claims 1 to 9 comprising the steps of:
(a) contacting a compound of formula (II) with a compound of formula (III) 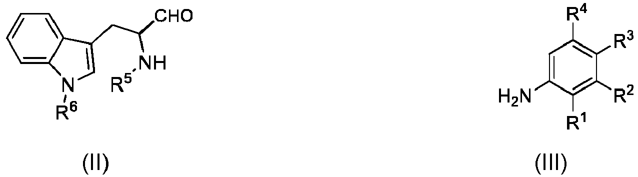 in order to form a compound of formula (IV) 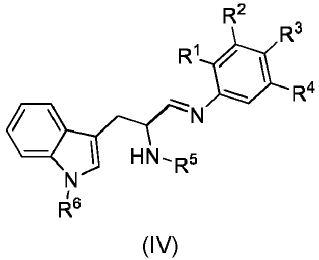 wherein R¹, R², R³, R⁴, R⁵ and R⁶ are as defined in any one of claims 1 to 9,
and (b) treating the product of step (a) with a cyanide source in order to form a product of formula (I).

14. Process according to claim 13 comprising the steps of:
(a) contacting a compound of formula (II) with a compound of formula (IIIa) 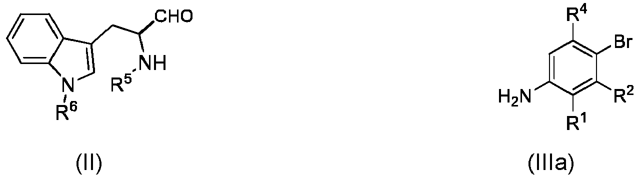 in order to form a compound of formula (IVa) 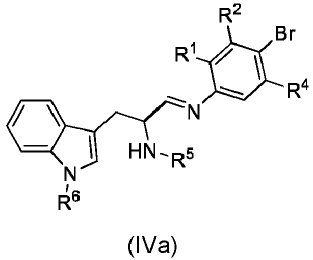 wherein R¹, R², R⁴, R⁵ and R⁶ are as defined in any one of claims 1 to 9,
(b) treating the product of step (a) with a cyanide source in order to form a product of formula (V) 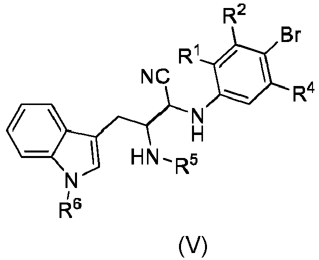
and (c) the step of contacting the compound of formula (V) with a compound of formula R³-B(OH)₂ in the presence of a catalytically effective amount of a cross-coupling catalyst, wherein R³ is a group selected from the group consisting of pyridinyl, pyrimidinyl and phenyl, said group being optionally substituted at any available position with one or two groups selected from the group consisting of halogen, (C₁-C₄)alkyl, (C₁-C₄)alkyloxy, trilfuoromethyloxy, di((C₁-C₄)alkyl)amino, phenyl, benzyl, (C₃-C₆)cycloalkyl and a 5 to 6-membered saturated heterocyclic ring, the members of the ring being selected from the group consisting of C, CH, CH₂, O, N and NH, said ring being optionally substituted at any available position with a (C₁-C₄)alkyl; or, alternatively, wherein said group selected from the group consisting of pyridinyl, pyrimidinyl and phenyl optionally forms a 6-membered ring with a divalent group of formula -O-(CH₂)₂-O- whereby the oxygen atoms of said divalent group are connected to two adjacent carbon atoms of said pyridinyl, pyrimidinyl or phenyl group.
